# DEMANDE DE BREVET EUROPEEN

(11) **EP 3 970 691 A1**
(43) Date de publication de la demande: **23.03.2022**
(21) Numéro de dépôt: 21197417.5
(22) Date de dépôt: 17.09.2021
(51) Int. Cl.: A61K 8/42, A61K 8/49, A61K 8/63, A61Q 19/06, A61K 8/9783

(54) **COMPOSITIONS COSMÉTIQUES AMINCISSANTES CONTENANT UN OU PLUSIEURS LIMONOÏDES**

(30) Priorité: 18.09.2020 FR 2009488
(71) Demandeur: C.F.E.B. Sisley, 75008 Paris (FR)
(72) Inventeur: THUILLIER, Isabelle, 75008 PARIS (FR); GINESTAR, José, 95800 COURDIMANCHE (FR); NADA, André, 78580 MAULE (FR); MAGNAN, Christophe, 75020 PARIS (FR); LE STUNFF, Hervé, 75012 PARIS (FR); LE MAREC, Axelle, DUBLIN, D03A8P7 (IE)
(74) Mandataire: Regimbeau

(57) **Abrégé**

La présente invention concerne l'utilisation cosmétique d'une composition contenant un ou plusieurs principes actifs ayant des effets amincissants et drainants. Ces principes actifs sont de préférence des limonoïdes tels que la gédunine et/ou la limonine. L'invention concerne également un procédé de soin cosmétique comprenant l'application topique, sur au moins une partie de la peau du corps ou du visage, d'une composition selon l'invention contenant des extraits végétaux riches en ce principe actif, ainsi qu'un milieu physiologiquement acceptable, afin d'obtenir un effet amincissant, plus précisément pour réduire l'excès de graisse corporelle localisé, affiner la silhouette, et réduire la présence de cellulite.

## Description

### Résumé de l'invention

La présente invention concerne l'utilisation cosmétique d'une composition contenant un ou plusieurs principes actifs ayant des effets amincissants et drainants. Ces principes actifs sont de préférence des limonoïdes tels que la gédunine et/ou la limonine. L'invention concerne également un procédé de soin cosmétique comprenant l'application topique, sur au moins une partie de la peau du corps ou du visage, d'une composition selon l'invention contenant des extraits végétaux riches en ce principe actif, ainsi qu'un milieu physiologiquement acceptable, afin d'obtenir un effet amincissant, plus précisément pour réduire l'excès de graisse corporelle localisé, affiner la silhouette, et réduire la présence de cellulite.

### Contexte de l'invention

Le tissu adipeux sous-cutané est situé au niveau de l'hypoderme. Dans ce tissu conjonctif prédominent les adipocytes, organisés en lobes d'environ 5 mm de diamètre, séparés par de fins ponts conjonctifs. Le tissu adipeux peut être considéré comme un réservoir dynamique, constamment renouvelé, équilibrant l'apport alimentaire avec les besoins énergétiques du corps. Ainsi, les adipocytes assurent la synthèse, l'accumulation et la libération des lipides.

La synthèse lipidique, ou lipogenèse, prend naissance avec des triglycérides d'origine alimentaire et du glucose. Inversement, les triglycérides stockés dans les adipocytes peuvent être hydrolysés, lors de la lipolyse, pour libérer des acides gras, du glycérol et des mono et diesters de glycérol.

Les acides gras non estérifiés ainsi libérés peuvent circuler dans le sang puis être disponibles pour les besoins énergétiques des autres cellules du corps, ou être rapidement réutilisés par l'adipocyte afin de générer, là encore, des triglycérides par lipogenèse. Si un déséquilibre durable se produit dans le corps favorisant la lipogenèse, la quantité de lipides stockés dans les adipocytes augmente, conduisant à une hyperplasie de la masse de graisse corporelle et plus précisément à l'apparition d'un excès de graisse corporelle localisé. L'excès de graisse corporelle localisé est souvent associé à des modifications de la peau, qui développe une apparence alvéolée ou "peau d'orange".

La structure anatomique du tissu adipeux sous-cutané est souvent considérée comme principale cause de cellulite. Les études histologiques des tissus sous-cutanés des hommes et des femmes suggèrent que les lobules graisseux sont plus grands et plus verticaux chez les femmes que chez les hommes. En conséquence, ces lobules plus gros et moins restreints peuvent s'exprimer vers l'extérieur contre le derme, provoquant les bosses et les fossettes caractéristiques de la cellulite. Les dépôts de graisse sous-cutanée fémorale chez la femme ont également tendance à être plus lipogéniques et moins lipolytiques que la graisse sous-cutanée ou viscérale abdominale en raison de la différence de distribution des récepteurs adrénergiques alpha et bêta sur les adipocytes dans ces différentes régions. Une lipolyse accrue ou une réduction de la graisse de ces sites adipeux sous-cutanés sélectionnés peut conduire à une réduction ou à la prévention de la cellulite.

De nombreux actifs ayant une action sur la lipolyse ou la lipogenèse, destinés à un effet amincissant, ont été identifiés. Parmi elles, on peut citer :
- Les dérivés de la xanthine, telles que la théophylline, la caféine, la théobromine, utilisées pour leur action favorisant l'activité lipolytique des cellules graisseuses
- Les peptides synthétiques, tels que le peptide de séquence Arg-Gly-Ser-NH₂ (décrit dans le brevet FR 2 858 769), ou le peptide de séquence Pro-Leu-Asp-Thr-Ala-Lys-Val-Arg-Leu-Gln (décrit dans le brevet FR 2 879 924) utilisé pour son action dans le découplage entre la réoxydation des coenzymes et la phosphorylation de l'ADP en ATP dans les mitochondries.
- Des extraits de plantes, comme l'extrait d'algues marines du genre Palmaria ou Rhodymenia (décrit dans le brevet FR 2 887 447), des extraits de Ginkgo biloba (décrit dans le brevet FR 2 669 537), ou des flavones de soja ou des isoflavones (décrits dans le brevet WO 01/64177).

Cependant, ces produits ont généralement une efficacité modérée ou limitée dans le temps. Il est donc important de proposer de nouveaux actifs cosmétiques ayant une efficacité remarquable comme actifs amincissants.

### Description détaillée de l'invention

Dans ce contexte, les présents inventeurs ont mis en évidence le rôle majeur que joue l'enzyme de transfert des céramides (ci-après appelée « enzyme CERT ») sur la différenciation adipocytaire. L'enzyme CERT transporte les céramides du réticulum endoplasmique où ils sont synthétisés vers l'appareil de Golgi où ils servent de base à la synthèse de sphingomyélines.

Les présents inventeurs ont étudié la capacité des cellules à se différencier en mesurant notamment le niveau d'expression de gènes impliqués dans l'adipogenèse et dans la fonction adipocytaire, la sécrétion d'adiponectine et la capacité de stockage de TG dans les cellules au terme de traitements avec des inhibiteurs de l'enzyme CERT tels que la gédunine et la limonine. Ils ont pu démontrer que, lorsque des cellules fibroblastiques adipocytaires sont traitées avec de tels inhibiteurs pendant leur maturation, leur capacité de différenciation est considérablement diminuée. Ceci suggère un fort effet anti-adipogénique de ces inhibiteurs. Ces résultats, obtenus sur des lignées cellulaires 3T3-L1, ont été confirmés sur des pré-adipocytes humains primaires issus de tissu adipeux sous-cutanés (exemple A).

Dans un premier aspect, la présente invention concerne une composition cosmétique à usage topique, ladite composition comprenant une quantité efficace d'au moins un inhibiteur de l'enzyme de transfert des céramides (CERT).

L'enzyme de transfert des céramides peut être inhibée par de nombreuses molécules de type limonoïde, comme cela est décrit dans Hullin-Matsuda et al, 2012. Elle peut également être inhibée par des petites molécules chimiques telles que HPA-12 et HPA-14 (Charruyer et al, 2008).

Les limonoïdes sont des tétranortriterpènes comportant en général 4 cycles de 6 atomes et un cycle de furane. Ils sont naturellement présents dans les agrumes et dans d'autres plantes de la famille des Rutacées et Méliacées.

De manière préférentielle, ledit inhibiteur de l'enzyme CERT est un limonoïde choisi parmi : la cedrelone, la khayanthone, l'odoratone, la gédunine, la khivorine, l'isogedunine, la limonine, la methylangolensate, l'obliquine, la prieuranine, et la carapine ou un de leurs sels ou dérivés acceptables.

De manière encore plus préférée, ledit inhibiteur de l'enzyme CERT est choisi parmi la gédunine et la limonine, ou l'un de leurs sels ou dérivés acceptables.

La gédunine a la formule chimique suivante :

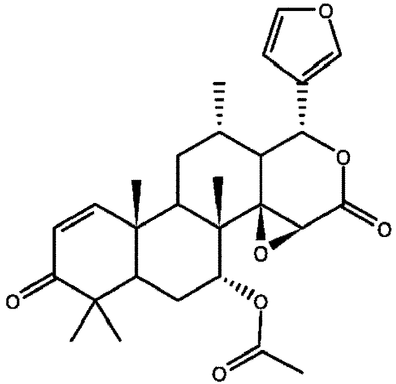

La limonine a la formule chimique suivante :

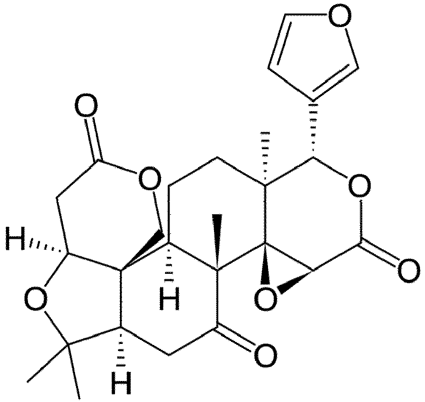

On entend ici désigner par « sels acceptables » des sels qui sont cosmétiquement acceptables et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Plus généralement, il est possible d'utiliser dans la composition de l'invention toute molécule capable d'inhiber efficacement l'enzyme CERT.

Par « inhiber efficacement l'enzyme CERT », on entend ici que les molécules actives de l'invention sont capables d'empêcher efficacement la fonction biologique de l'enzyme CERT (Ceramide transporter), qui consiste à extraire les céramides de la membrane du réticulum endoplasmique et de les transporter dans l'appareil de Golgi. En d'autres termes, les molécules actives de l'invention sont capables de diminuer significativement le transport des céramides dans l'appareil de Golgi. Cette inhibition peut être mesurée par exemple par immunohistochimie en marquant les céramides et en analysant leur localisation à l'intérieur de cellules traitées par la molécule testée (Hullin-Matsuda et al, 2012), ou en utilisant une méthode enzymatique, par exemple celle impliquant la DAG kinase (cette enzyme est capable de phosphoryler les céramides avec de l'ATP radiomarqué, la radioactivité incorporée est ensuite mesurée dans les échantillons, de sorte à doser la quantité totale de céramides présents dans les échantillons).

Deux molécules inhibitrices de CERT sont ici particulièrement préférées. Il s'agit de la gédunine et de la limonine. Leur capacité à inhiber l'enzyme CERT est bien connue (Hullin-Matsuda et al, 2012). Les mesures réalisées in silico par les inventeurs ont permis de déterminer que la gédunine interagit, tout comme HPA-14, avec les acides aminés Tyr553 et Gln467 de la CERT, avec un Ki très bas, de 3,93 nM (exemple A). De même, ils ont pu montrer que la limonine possède trois sites d'interaction avec CERT communs avec la gédunine, et se fixe à la CERT avec un Ki de 54,08 nM. Les inventeurs ont également démontré que la gédunine et la limonine ont un effet significatif sur les cellules adipocytaires (exemple A et exemple B).

Les composés selon l'invention possèdent tous plusieurs centres d'asymétrie et peuvent donc exister sous forme d'isomères optiques. La présente invention vise aussi bien ces isomères soit séparément soit en tant que mélange, désignés ci-après sous le terme « dérivés ». Dans la présente invention, on entend désigner par « isomères » des composés qui ont des formules moléculaires identiques mais qui diffèrent par l'agencement de leurs atomes dans l'espace. Les isomères qui diffèrent dans l'agencement de leurs atomes dans l'espace sont désignés par « stéréoisomères ». Les stéréoisomères qui ne sont pas des images dans un miroir l'un de l'autre sont désignés par « diastéréoisomères », et les stéréoisomères qui sont des images dans un miroir non superposables sont désignés par « énantiomères », ou quelquefois isomères optiques. Un atome de carbone lié à quatre substituants non identiques est appelé un « centre chiral ».

Le terme « isomère chiral » caractérise un composé avec un centre chiral. Il comporte deux formes énantiomères de chiralité opposée et peut exister soit sous forme d'énantiomère individuel, soit sous forme de mélange d'énantiomères. Un mélange contenant des quantités égales de formes énantiomères individuelles de chiralité opposée est désigné par «mélange racémique».

Le terme « conformère » désigne ici un stéréoisomère dans lequel les isomères peuvent être convertis par rotation autour de liaisons simples.

Les « dérivés acceptables » visés dans la présente invention sont de préférence des isomères (par exemple des stéréoisomères - diastéréoisomères ou énantiomères » - ou des conformères) des composés décrits dans la présente demande.

Ces dérivés peuvent être également des triterpènes dérivés des composés de l'invention, par exemple dérivés de la gédunine ou de la limonine, possédant l'activité pharmacologique souhaitée du composé parent.

Dans un mode de réalisation préféré, la composition de l'invention comprend une quantité efficace de gédunine et/ou une quantité efficace de limonine, ou de l'un de leur dérivés.

La détermination d'une « quantité efficace » d'au moins un inhibiteur selon l'invention est à la portée de l'homme du métier. Cette quantité se réfère à la quantité d'inhibiteur, par exemple, d'un constituant limonoïde identifié conformément à la présente invention, qui, par exemple, améliore, réduit ou élimine la condition, plus spécifiquement, la cellulite.

A titre d'exemple, la composition cosmétique de l'invention peut avantageusement contenir au moins 0.01 ppm de limonine et/ou au moins 5 ppm de gédunine. De préférence, elle contient au moins 0.04 ppm de limonine et/ou au moins 9 ppm de gédunine.

Lorsque lesdits limonoïdes sont introduits dans la composition en tant que molécules synthétiques, la composition de l'invention peut éventuellement ne pas contenir d'autres agents actifs ayant des propriétés amincissantes.

Les limonoïdes contenus dans les compositions de la présente invention peuvent être synthétisés chimiquement à l'échelle industrielle en grandes quantités. Alternativement, ces limonoïdes peuvent être extraits des matières premières naturelles des plantes.

La composition de l'invention comprend avantageusement au moins 0,5 %, de préférence au moins 1%, d'un extrait de plante contenant au moins 1000ppm d'inhibiteurs de CERT, par exemple d'un extrait de plante contenant au moins 1000ppm d'un limonoïde, par exemple au moins 1000 ppm de gédunine et/ou d'un de ses dérivés, ou d'un extrait de plante contenant au moins 4ppm, de préférence 15ppm, d'un limonoïde tel que la limonine.

La composition selon l'invention peut donc contenir par exemple au moins 0,5 % d'un extrait de plante contenant au moins 1000ppm de gédunine et/ou d'un de ses dérivés ou au moins 0,5 % d'un extrait de plante contenant au moins 4 ppm, de préférence 15 ppm, de limonine et/ou d'un de ses dérivés.

La composition selon l'invention peut également contenir par exemple au moins 1% d'un extrait de plante contenant au moins 1000ppm de gédunine et/ou d'un de ses dérivés et au moins 1 % d'un extrait de plante contenant au moins 4 ppm, de préférence 15 ppm, de limonine et/ou d'un de ses dérivés.

Selon un mode de réalisation avantageux, la composition de l'invention comprend entre 0,5% et 5%, de préférence entre 1% et 2% d'un extrait de plante contenant au moins 1000ppm de gédunine et/ou d'un de ses dérivés ou entre 0,5% et 5%, de préférence entre 1% et 2% d'un extrait de plante contenant au moins 4 ppm, de préférence 15 ppm, de limonine et/ou d'un de ses dérivés.

Selon un autre mode de réalisation avantageux, la composition de l'invention comprend entre 0,5% et 5%, de préférence entre 1% et 2% d'un extrait de plante contenant au moins 1000ppm de gédunine et/ou d'un de ses dérivés et entre 0,5% et 5%, de préférence entre 1% et 2% d'un extrait de plante contenant au moins 4 ppm, de préférence 15 ppm, de limonine et/ou d'un de ses dérivés.

La composition de l'invention contient avantageusement des extraits de plante riches en limonoïdes. Les plantes riches en limonoïdes ont été décrites dans la littérature (Roy et al, 2006). Ces plantes sont par exemple : le Margousier, l'Andiroba, le Khaya, le Yucca, le Munronia, le Cedrela, le Citrus nobilis, l'Helietta apiculata, la Mentha arvensis, la Cipadessa baccifera, le Xylocarpus, etc.

Plus précisément, il est connu que les parties de plantes suivantes contiennent de grandes quantités de limonoïdes :
- écorce de CEDRELA ODORATA et de CEDRELA GRAZIOVII
- graines de KHAYA SENEGALENSIS ou CAÏLCÉDRAT ou ACAJOU DU SÉNÉGAL
- graines de TRICHILIA CATIGUA
- écorce de WALSURA YUNNANENSIS
- écorces de tiges et de racines de TURRAEA HOLSTII
- bois, graines, écorces de CEDRELA TOONA
- feuilles de TRICHILIA AMERICANA
- rameaux et feuilles de TOONA CILIATA
- fruits de WALSURA ROBUSTA
- bois de TOONA AUSTRALIS
- graines de KHAYA ANTHOTHECA
- écorces de KHAYA IVORENSIS, MITRAGYNA STIPULOSA et KIGELA AFRICANA
- écorce d'HELIETTA APICULATA
- huile d'ANDIROBA (CARAPA GUIANENSIS, MELIACEAE)
- huile et fruits et noyaux de AZADIRACHTA INDICA
- écorce d'ENTANDROPHRAGM ANGOLENSE
- écorce et grains de KHAYA GRANDIFOLIOLA
- pistils de CEDRELA ODORATA greffé sur TOONA CILIATA VAR. AUSTRALIS
- écorce de T. CATIGUA A. JUSS.
- branche de CARAPA GUIANENSIS AUBL. (MELIACEAE)
- bois de XYLOCARPUS RUMPH II
- graines de XYLOCARPUS GRANATUM
- écorce de CEDRELOPSIS GREVEI
- écorce, pulpe, pépins de CITRUS NOBILIS

Tout extrait de ces plantes, contenant une quantité efficace de limonoïde, en particulier de la gédunine et/ou de limonine au sens de l'invention, peut être utilisé dans la composition de l'invention.

Il est possible d'utiliser chacune d'elles dans les proportions qui permettent d'obtenir une quantité efficace d'inhibiteur, notamment de gédunine ou de limonine. De préférence, des extraits de plante riche en limonoïdes seront donc incorporés dans la composition de l'invention.

Par «extrait de plante», on entend toute substance ou préparation isolée extraite d'une plante, indépendamment de la méthode d'extraction utilisée. Le terme est utilisé au sens large, comprenant, par exemple, des ingrédients solubles dans l'eau ou des ingrédients organiques extraits à l'aide du solvant, ou des ingrédients spécifiques de la plante.

Toutes les méthodes de préparation peuvent être utilisées, y compris l'utilisation de cellules de semences de plantes cultivées. Le niveau d'extraction et le degré de pureté du limonoïde peuvent varier. Par exemple, des extraits de plantes non purifiés peuvent être utilisés dans la présente invention. Selon la solubilité du limonoïde particulier dans l'eau ou dans un solvant organique, leur processus d'extraction peut différer. Deux méthodes d'extraction possibles sont l'extraction par solvant organique et l'extraction par solvant aqueux-organique, comme décrit dans le brevet US 6,372,239 de Wu et al. La méthode d'extraction organique implique une étape de lavage et d'extraction continue du matériel végétal contre un courant de solvant organique. Des exemples de solvants organiques comprennent, mais sans s'y limiter, le méthanol, l'éthanol, le dichlorométhane, le chloroforme, le xylène et l'éther de pétrole. Alternativement, le limonoïde peut être partiellement purifié ou complètement purifié.

De préférence, la composition de l'invention contient un extrait d'Andiroba car cette plante est connue pour contenir de fortes quantités de gédunine. Par exemple, la composition de l'invention peut contenir de l'huile d'Andiroba obtenue par une méthode d'extraction conservant de fortes quantités de gédunine. Il existe de nombreux documents décrivant de tels extraits d'Andiroba et les méthodes pour les produire (cf. WO 2006/007680). De manière encore plus préférée, l'huile d'Andiroba utilisée dans la composition de l'invention est obtenue en extrayant de manière mécanique de l'huile à partir de graines d'Andiroba, cette huile étant par la suite raffinée. Il est possible d'utiliser notamment l'huile d'Andiroba commercialisée par Expanscience, qui a été obtenue par extraction mécanique à partir de graines d'Andiroba.

De préférence, la composition de l'invention contient un extrait de Citrus nobilis (autrement appelé « mandarinier »), produit de manière à contenir de fortes quantités de limonine. Cet extrait doit avoir été obtenu par une méthode d'extraction conservant de fortes quantités de limonine, par exemple entre 4 et 100ppm. Il existe de nombreux documents décrivant de tels extraits de Citrus, et les méthodes pour les produire (cf. par exemple l'article de Setyadjit et al, 2013). De manière encore plus préférée, l'extrait de mandarine utilisé dans la composition de l'invention est obtenu en utilisant la technique d'extraction par CO₂ supercritique :

L'extraction par CO₂ supercritique consiste à faire circuler du CO₂ supercritique, sous pression et température, à travers une matière, puis d'opérer une décompression pour récupérer l'extrait. A la dépressurisation, le CO₂ est libéré sous forme gazeuse (ré-exploitable) et le composé recherché sous forme liquide ou solide. Le CO₂ supercritique peut voir sa polarité modifiée par l'ajout d'un « dopant » polaire communément appelé co-solvant permettant ainsi d'augmenter le spectre des molécules extraites par le solvant supercritique.

Il est par exemple possible d'utiliser l'extrait de mandarine « Citrus Nobilis extract » commercialisé par Sederma, et obtenu par extraction avec du CO₂ supercritique, cet extrait contenant au minimum 4ppm de limonine.

Dans cette composition, l'extrait de plante contenant la gédunine est de préférence de l'huile d'Andiroba décrite ci-dessus et l'extrait de plante contenant la limonine est de préférence l'extrait de mandarine décrit ci-dessus.

Tel qu'utilisé dans la présente demande, le terme « % » fait référence au « % en poids », c'est-à-dire au pourcentage en poids d'un composant par rapport au poids total de la composition (c'est-à-dire, y compris tous supports, véhicules, solvants, charges ou autres composants ajoutés avant l'application sur la peau), sauf disposition contraire.

Les compositions topiques de l'invention peuvent notamment se présenter sous la forme d'une solution aqueuse, hydro-alcoolique ou huileuse; d'une émulsion huile-dans-eau, eau-dans-huile ou d'une émulsion multiple; elles peuvent également se présenter sous forme de suspensions, ou de poudres, adaptées à une application sur la peau ou sur les muqueuses.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent également se présenter sous forme solide comme un stick ou être appliquées sur la peau sous forme d'aérosol.

La composition cosmétique de l'invention contient outre l'inhibiteur de CERT, de préférence au moins un excipient dermatologiquement acceptable. Cet excipient peut être, par exemple, un excipient utilisé habituellement dans les compositions topiques destinées au soin de la peau.

Dans le contexte de la présente invention, le terme "excipient dermatologiquement acceptable" signifie que ledit excipient convient à une utilisation en contact avec la peau humaine sans toxicité, incompatibilité, instabilité, irritabilité, réponse allergique, etc. Les excipients appropriés comprennent, par exemple, les huiles minérales et les agents émulsifiants. Dans son mode de réalisation le plus simple, le support peut être de l'eau, de l'alcool ou des combinaisons eau / alcool, ou d'autres solvants ou systèmes de solvants dans lesquels les principes actifs susmentionnés peuvent être, par exemple, solubles, dispersés, émulsionnés, etc. La composition de l'invention comprend, de préférence, un ou plusieurs excipients qui permet à la composition d'être uniformément répartie sur la peau et / ou fournissent du corps et de la viscosité à la composition de l'invention, de sorte que les principes actifs pénètrent bien dans la peau, une fois la composition appliquée. Typiquement, l'excipient constitue entre environ 30 et environ 99% en poids de la composition cosmétique de l'invention.

Des excipients particuliers sont décrits en détail dans, par exemple, Gonzalez et. Al. - US 7 186 404; Aust et. Al. - US 7 175 834; Roseaver et. al.- US 7 172 754; Simoulidis et. Al. - US 7 175 835; Mongiat et. Al. - US 7 101 536; Maniscalco - US 7 078 022. L'homme du métier reconnaîtra et appréciera facilement quels excipients peuvent être utilisés en fonction de la forme et/ou du mode d'administration de la composition de l'invention.

La composition cosmétique de l'invention peut être formulée de préférence sous forme d'émulsion, pour une application topique, sur la peau.

De préférence, l'émulsion de l'invention contient, comme excipient, de l'eau, des solvants organiques miscibles à l'eau (comme les alcools), des agents gélifiants/stabilisants (tels que des gommes, des polymères synthétiques et leurs dérivés), des corps gras liquides (tels que les huiles volatiles ou non volatiles), des corps gras solides tels que les cires, les corps gras pâteux, les gommes et leurs mélanges, des agents tensioactifs émulsionnants parmi des agents tensioactifs anioniques, cationiques, amphotères ou non ioniques, une phase particulaire additionnelle pouvant notamment comprendre au moins un pigment et/ou au moins une nacre et/ou au moins une charge complémentaire, des colorants hydrosolubles ou liposolubles, des parfums ou bien des agents alcalinisants ou acidifiants, des agents antioxydants, ou des conservateurs ou un autre excipient connu, ou leur mélange.

Dans tous les cas, l'homme du métier veillera à ce que lesdits excipients ainsi que leurs proportions soient choisis de manière à ne pas interférer avec les propriétés avantageuses recherchées de la composition de l'invention. Ces excipients peuvent par exemple correspondre à 0,01 à 20% du poids total de la composition. Lorsque la composition de l'invention est une émulsion, la phase grasse peut représenter 5 à 80% en poids et de préférence 5 à 50% en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30% en poids, par rapport au poids total de la composition.

Avantageusement, la composition de l'invention peut comprendre, en plus de l'inhibiteur de CERT, au moins un autre agent actif ayant des effets cosmétiques similaires et / ou complémentaires à ceux de l'invention. Selon l'invention, cet agent actif sera défini comme un "agent actif supplémentaire".

Par exemple, ledit agent actif supplémentaire peut être choisi parmi les agents anti-âge, tonifiants, éclaircissants, hydratants, drainants, agents favorisant la microcirculation, exfoliants, gommants, agents stimulant la matrice extracellulaire, agents activant le métabolisme énergétique, agents antibactériens, antifongiques, calmants, anti-radicaux libres, anti-UV et anti-acnéiques, anti-inflammatoires, anesthésiques, réchauffants, rafraîchissants et autres agents amincissants.

Plus particulièrement, cet agent actif peut être par exemple choisi parmi : les agents antiglycation et/ou antioxydants pris seuls ou en association (tels que par exemple le tocophérol et ses dérivés, l'ergothionéine, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate), les agents anti-inflammatoires (tels que par exemple l'alpha-bisabolol ou le stearyl glycyrrhetinate), les agents apaisants et leurs mélanges, les agents conservateurs (anti-bactériens ou anti-fongiques), les agents hydratants, les modificateurs de pH, les agents kératolytiques et/ou desquamants (tels que par exemple l'acide salicylique et ses dérivés), les vitamines, les épaississants, les agents émollients, les eaux thermales, les tensioactifs, les polymères, les huiles de silicone, les huiles végétales, les huiles essentielles, les parfums, les matières colorantes ou les pigments, etc.

De manière particulièrement avantageuse, ledit agent actif supplémentaire peut avoir un effet amincissant, inhibant la lipogenèse ou stimulant la lipolyse. Il peut être choisi parmi les composés suivants: AMP cyclique et ses dérivés, agents activateurs d'enzyme adénylate cyclase et agents inhibiteurs d'enzyme phosphodiestérase, extrait de Centella asiatica, asiaticoside et acide asiatique, méthylxanthines, caféine, théine, théophylline, théobromine, forskoline, esculine et esculoside, inhibiteurs de l'ECA, peptide Val-Trp, inhibiteurs du neuropeptide Y, enképhaline, extrait de Ginkgo biloba, dioscorée, rutine, extrait de Verba mate, extrait de guarana, oligosaccharides, polysaccharides, carnitine, extrait de lierre, extrait de Prunella vulgaris hydrolysé, extrait de Celosia cristata hydrolysé, extrait d'Anogeissus leiocarpus, extrait de feuille de Manihot utilissima, palmitoylcarnitine, carnosine, taurine, extrait de sureau, extraits d'algues, extrait de palmier, peptide synthétique de séquence Arg-Gly-Ser-Nh2, vendu sous la dénomination ATPeptide^{™}, le peptide synthétique de séquence Pro-Leu-Asp-Thr-Ala-Lys-Val-Arg-Leu-Gln vendu sous la dénomination UCPeptide^{™}.

Les compositions de l'invention peuvent comprendre tout additif couramment utilisé en cosmétique, ainsi que tous les adjuvants nécessaires à leur formulation, tels que solvants, épaississants, diluants, antioxydants, colorants, filtres solaires, agents autobronzants, pigments, charges, conservateurs, parfums, absorbeurs d'odeurs, actifs cosmétiques, huiles essentielles, vitamines, acides gras essentiels, tensioactifs, polymères filmogènes, etc.

Avantageusement, les compositions selon l'invention se présentent sous une forme adaptée à une application topique. Dans ce cas, elles doivent contenir un milieu physiologiquement acceptable, c'est-à-dire compatible avec la peau et les appendices cutanés, et couvrir toutes les formes cosmétiques décrites ci-dessus.

Le terme « physiologiquement acceptable » fait référence ici à tout composé adapté pour entrer en contact avec la peau ou une muqueuse sans provoquer de réactions de toxicité ou d'intolérance.

En règle générale, les compositions cosmétiques de l'invention peuvent être administrées une à deux fois par jour, tous les jours. Les variations de ces niveaux de dosage peuvent être ajustées en utilisant des routines empiriques standard pour l'optimisation, comme cela est bien compris dans l'art. Des indications quant aux dosages particuliers et aux méthodes d'administration sont fournies dans la littérature et sont généralement disponibles pour les praticiens de l'art. L'homme du métier emploiera différentes formulations en fonction de la nature, par exemple, de la structure, de la composition, du constituant limonoïde actif.

La composition de l'invention est destinée aux personnes en bonne santé qui souhaitent améliorer l'apparence de leur peau et de leur silhouette, réduire l'excès de graisse corporelle localisé à l'aide de méthodes cosmétiques.

Dans un second aspect, la présente invention concerne l'utilisation topique d'une composition cosmétique comprenant une quantité efficace d'au moins un inhibiteur de l'enzyme de transfert des céramides (CERT) pour prévenir ou diminuer la cellulite, et/ou pour promouvoir un effet amincissant.

La présente invention concerne également un procédé pour augmenter la lipolyse, amincir et drainer en utilisant une composition cosmétique de l'invention.

Toutes les caractéristiques présentées ci-dessus pour la composition de l'invention sont ici incluses, sans avoir besoin d'être répétées.

Ladite composition cosmétique comprend par exemple au moins 0.01 ppm de limonine et/ou au moins 5 ppm de gédunine. De préférence, elle contient au moins 0.04 ppm de limonine et/ou au moins 9 ppm de gédunine.

Alternativement, ladite composition cosmétique peut comprendre par exemple au moins 1 % d'un extrait de plante contenant au moins 1000ppm de gédunine et/ou d'un de ses dérivés et au moins 1 % d'un extrait de plante contenant au moins 4 ppm, de préférence 15 ppm, de limonine et/ou d'un de ses dérivés.

Ladite composition cosmétique peut comprendre plus particulièrement une quantité efficace d'un extrait d'Andiroba et une quantité efficace d'un extrait de mandarine, soit par exemple au moins 0,5 % d'huile d'Andiroba contenant au moins 1000ppm de gédunine et/ou un de ses dérivés et au moins 0,5 % d'un extrait de mandarine contenant au moins 4 ppm, de préférence 15 ppm, de limonine et/ou d'un de ses dérivés.

La présente invention concerne enfin une méthode de traitement cosmétique de la cellulite, comprenant l'application de la composition décrite ci-dessus, sur la peau d'un individu sain. Les inventeurs ont en effet démontré que l'administration régulière d'une composition selon l'invention sur la peau de sujets sains présentant de la cellulite, permettait de réduire l'aspect inesthétique de celle-ci, de lisser la peau, et de remodeler les parties du corps concernées (exemple C).

La présente invention vise donc également l'utilisation cosmétique d'un limonoïde tel que la gédunine ou la limonine comme actif amincissant. Plus particulièrement, elle vise l'utilisation d'une composition contenant au moins 0.01 ppm de limonine et/ou au moins 5 ppm de gédunine, de préférence au moins 0.04 ppm de limonine et/ou au moins 9 ppm de gédunine, comme actif amincissant. Lorsque lesdits limonoïdes sont utilisés en tant que molécules synthétiques, il est possible de les utiliser comme seuls agents actifs amincissants.

Utilisé selon l'invention, l'ensemble de la formule contenant ledit limonoïde offre en effet notamment les avantages suivants:
- Il lisse la peau et le microrelief de la peau, diminuant l'aspect "peau d'orange",
- Il réduit l'excès de dépôts graisseux localisés, modelant et raffermissant la peau traitée
- Il rétablit le flux sanguin et limite la stase, améliore la microcirculation cutanée, réduit la rétention d'eau, drainant la zone traitée.

La méthode cosmétique de l'invention ainsi que la composition cosmétique de l'invention, présentent donc tous ces avantages.

Par "agent actif amincissant" on entend ici un agent permettant de réduire un excès de dépôt de graisse localisé, considéré comme peu attrayant et souvent associé à une apparence alvéolée de la peau.

Le terme "élimination des lipides" se réfère au phénomène de lipolyse conduisant à l'exportation de glycérol de la cellule adipocytaire.

Le terme "cellulite" définit une apparence alvéolée de la peau, provoquée par des cellules graisseuses poussant contre le tissu conjonctif du corps. Le mot "cellulite" se réfère uniquement à l'apparence de la peau et ne décrit pas une condition médicale. La cellulite est associée aux bosses, bosses et fossettes qui apparaissent sur des zones spécifiques (par exemple les cuisses) de nombreux hommes et femmes. La cellulite affecte principalement les cuisses et les fesses, mais peut également être présente sur l'estomac et le haut des bras. Les symptômes de la cellulite ne sont pas pathologiques.

Le terme "drainage" au sens de l'invention désigne une meilleure microcirculation sanguine et lymphatique, une réduction de la rétention d'eau, une réduction du gonflement des jambes, une réduction de la sensation de jambes lourdes.

Le terme "application topique" désigne l'application ou l'étalement de la composition selon l'invention, à la surface de la peau ou d'une muqueuse.

L'expression "dépôt de graisse localisé en excès" se réfère à une zone hypertrophiée du tissu adipeux sous-cutané, qui peut avoir un aspect "peau d'orange".

Par "amincissement" ou "réduction d'un excès de poids localisé", on entend selon la présente invention une action pour éviter ou au moins réduire la formation de graisse sous-cutanée comme décrit ci-dessus. Cette action se traduit notamment par une diminution des excès ou des réserves disgracieuses, un affinement de la silhouette en accélérant l'élimination des excès, en définissant mieux le contour du corps ou une silhouette remodelée.

Aux fins de la présente invention, sauf limitation supplémentaire, le terme "réduire" signifie diminuer le volume, la taille, la masse, la densité, la quantité et / ou la qualité d'une substance. Par exemple, la réduction des graisses peut inclure la réduction de la quantité de nouvelles cellules graisseuses (par exemple, le nombre de nouvelles cellules graisseuses), la réduction du volume des cellules graisseuses, la réduction de la maturation des cellules graisseuses et / ou la dédifférenciation des cellules graisseuses.

On s'attend en outre à ce que l'invention soit utile pour des individus présentant des dépôts de graisse proéminents ou indésirables sur l'abdomen, la poitrine, les fesses, les hanches, les cuisses, les jambes, les genoux, les bras, le menton, le visage ou le cou.

Il est évident que l'invention concerne les mammifères en général, et plus spécifiquement les êtres humains.

De préférence, lesdits individus sont sains (c'est-à-dire qu'ils ne souffrent d'aucune maladie déclarée).

### Légendes des figures

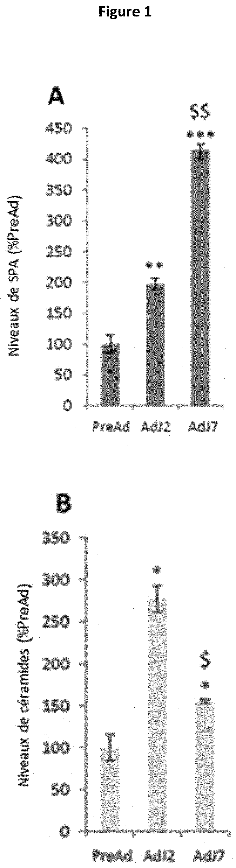

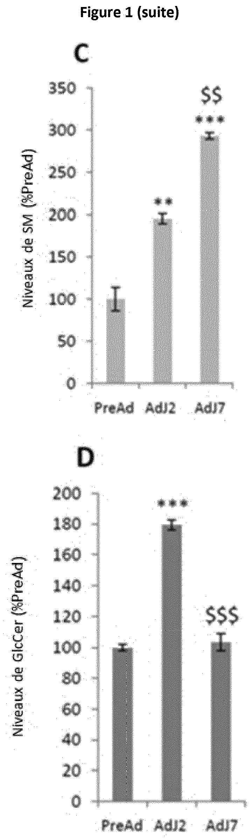

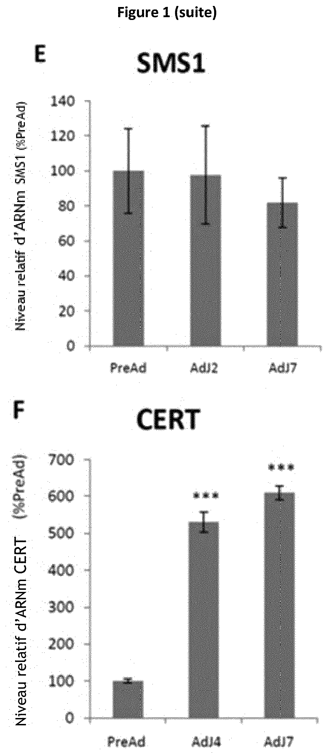

La **figure 1** représente l'analyse lipidomique au cours de la différenciation de cellules 3T3-L1 et les niveaux d'expression de gènes codant des protéines impliquées dans la synthèse des SM. « PreAd » représente les cellules 3T3-L1 non-différenciées ; « AdJ2 » corresponds aux cellules exposées au cocktail d'induction pendant deux jours ; « AdJ7 » représente les cellules exposées au milieu d'induction puis au milieu d'accumulation pendant 5 jours.
**A :** Quantité de sphinganine au cours de la différenciation exprimée en pourcentage des préadipocytes;
**B** : Quantité de céramides en pourcentage des préadipocytes ;
**C :** Quantité des sphingomyéline en pourcentage des préadipocytes ;
** = p ≤ 0,05 ; ** = p ≤ 0,01 ; *** = p ≤ 0,001 par rapport aux PreAd ; $ = p ≤ 0,05 ; $$ = p ≤ 0,01 ; $$$ =*
*p ≤ 0,001 par rapport aux AdJ2.D* : Quantité des glucosylcéramides en pourcentage des préadipocytes.
* = p ≤ 0,05 ; ** = p ≤ 0,01 ; *** = p ≤ 0,001 par rapport aux préadipocytes. $ = p ≤ 0,05; $$ = p ≤ 0,01 ; $$$ = p ≤ 0,001 par rapport aux AdJ2 (t test) n = 4.
**E** : Niveau d'expression de la sphingomyéline synthase 1 au cours de la différenciation (exprimé en pourcentage des préadipocytes) ;
**F :** Niveau d'expression du transporteur CERT au cours de la différenciation (exprimé en pourcentage des préadipocytes) ; *** = p ≤ 0,001 par rapport aux préadipocytes (t test) n = 4.

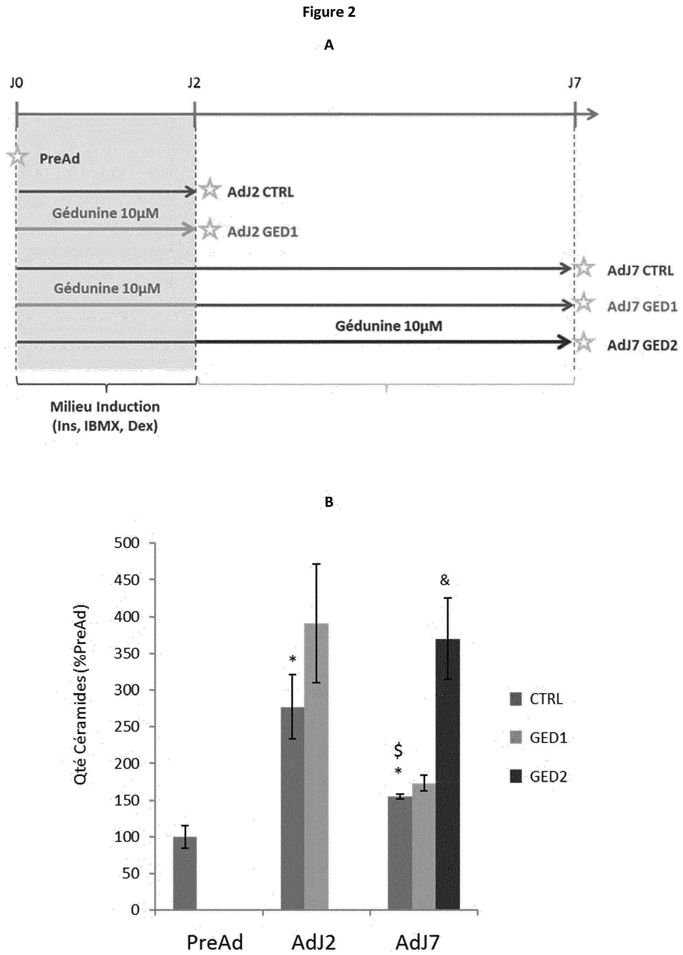

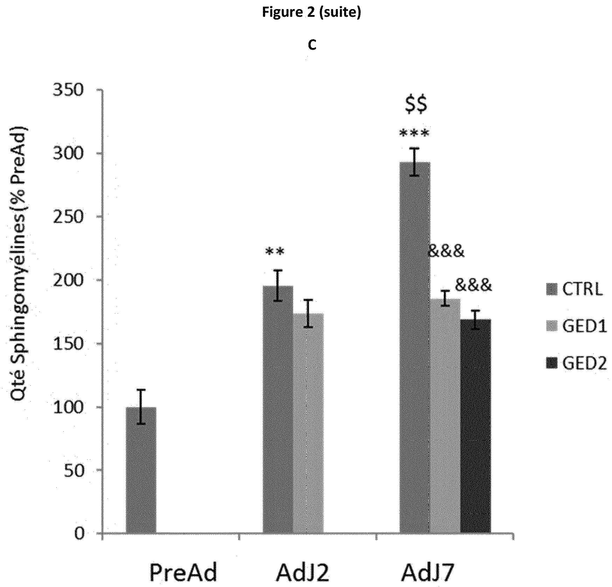

La **figure 2** décrit en **A** le protocole de différenciation des 3T3-L1 et timing des différents traitements à la Gédunine et en **B** et **C** les effets d'un traitement à la Gédunine sur les quantités de céramides totaux et des sphingomyélines au cours de la différenciation. Les cellules 3T3-L1 sont exposées ou non à la Gédunine à 10µM au cours de leur protocole de différenciation. Les échantillons « GED1 » ont été traités à la Gédunine 10µM pendant la phase d'induction, les échantillons « GED2 » ont été exposés à la Gédunine 10µM pendant la phase d'accumulation. **B** : Dosage des céramides totaux exprimés en pourcentage des préadipocytes ; **C** : Dosage des sphingomyélines, en pourcentage des préadipocytes. * = p ≤ *0,05 ;* ** = p ≤ 0,01 ; *** = p ≤ 0,001 par rapport aux préadipocytes. $ = p ≤ 0,05; $$ = p ≤ 0,01 par rapport aux AdJ2 CTRL. &&& = p ≤ 0,001 par rapport aux AdJ7 CTRL (t test) n = 4.

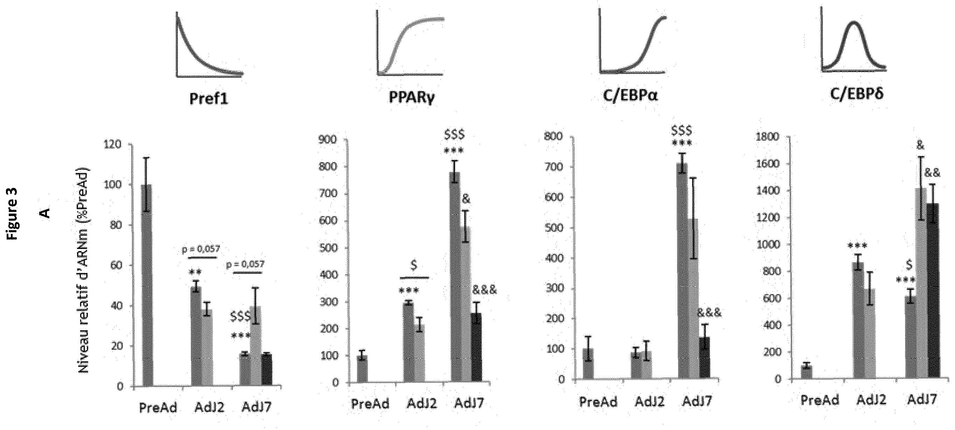

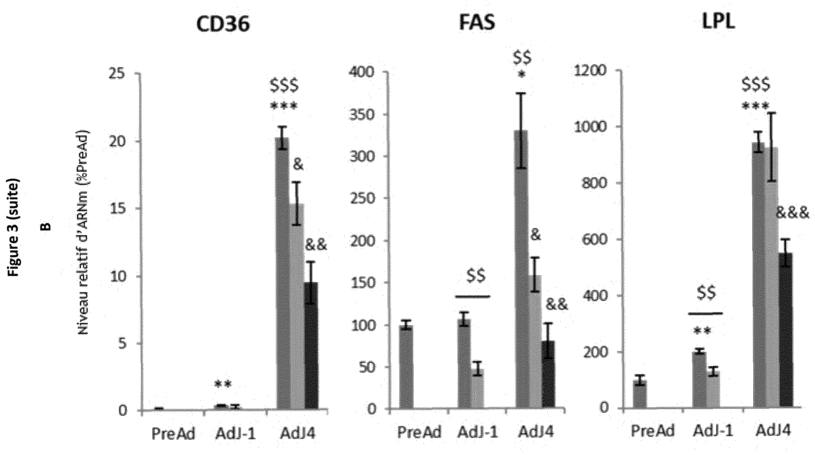

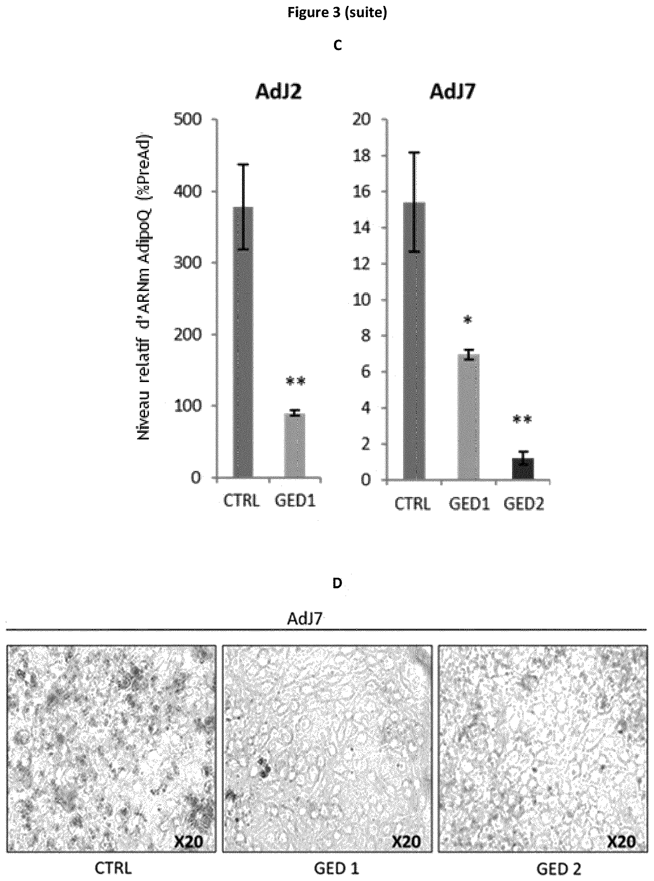

La **figure 3** démontre les effets de traitements à la Gédunine au cours de la phase d'induction ou de la phase d'accumulation du protocole de différenciation des 3T3-L1 sur la capacité de différenciation des cellules. Les échantillons « GED1 » ont été traités à la Gédunine 10µM durant la phase d'induction et les échantillons « GED2 » ont été traités à la Gédunine 10µM pendant la phase d'accumulation. Pour chaque condition (PreAd, AdJ2 et AdJ7), les trois colonnes, de gauche à droite, représentent respectivement les valeurs contrôle, GED1 et GED2.

**A :** Niveaux d'expression de gènes impliqués dans l'adipogénèse, exprimés en pourcentage des niveaux d'expression chez les préadipocytes ; **B** : Niveaux d'expression de gènes impliqués dans la fonction adipocytaire, exprimés en pourcentage des niveaux d'expression chez les préadipocytes ; C : Niveaux d'expression du gène codant l'adiponectine aux stades AdJ2 et AdJ7 ; * = p ≤ 0,05 ; ** = p ≤ 0,01 ; *** = p ≤ 0,001 par rapport aux préadipocytes. $ = p ≤ 0,05 ; $$ = p ≤ 0,01 ; $$$ = p ≤ 0,001 par rapport au CTRL AdJ2. & = p ≤ 0,05 ; && = p ≤ 0,01 ; &&& = p ≤ 0,001 par rapport au CTRL AdJ7 (t test) n = 4.; **D** : Colorations Huile Rouge observées en microscopie au grossissement x20.

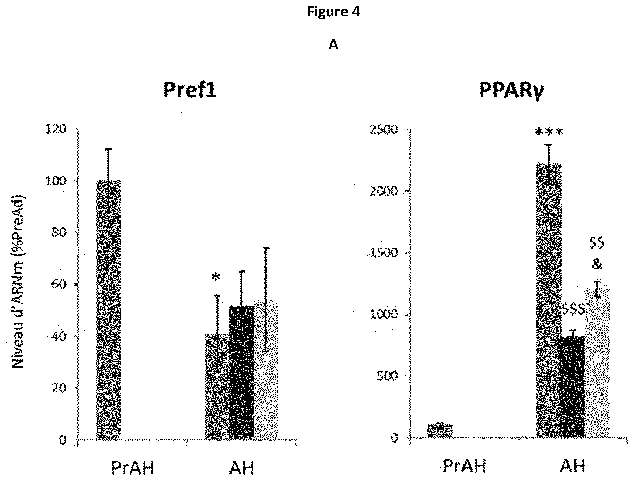

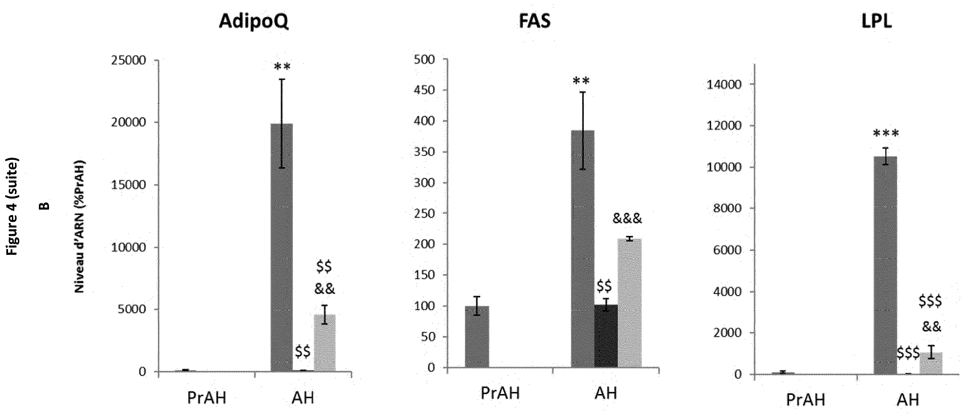

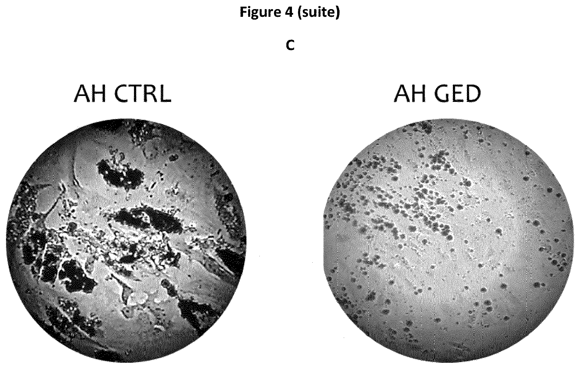

La **figure 4** décrit l'effet d'un traitement à la Gédunine sur la capacité de différenciation de préadipocytes humains *in vitro.* **A** : Niveaux d'expression des gènes PPARγ et Pref1 impliqués dans l'adipogénèse exprimés en pourcentage des préadipocytes. Colonnes de gauche à droite : contrôle, GED, rescue. **B:** Niveaux d'expression de gènes codant des protéines nécessaires à la fonction adipocytaire, exprimés en pourcentage des préadipocytes. * = p ≤ 0,05 ; ** = p ≤ 0,01 ; *** = p ≤ 0,001 par rapport aux préadipocytes. $$ = p ≤ 0,01 ; $$$ = p ≤ 0,001 par rapport au contrôle différencié. & = p ≤ 0,05 ; && = p ≤ 0,01 ; &&& = p ≤ 0,001 par rapport aux cellules traitées à la gédunine. Colonnes de gauche à droite : contrôle, GED, rescue. **C** : Colorations Huile Rouge observées en microscopie au grossissement x10.

La **figure 5** décrit le pourcentage de changement des paramètres d'ondulation (Ra et Rz) évalués dans deux zones de la cuisse, une avec cellulite et une autre sans cellulite, à J14 et à J28 par rapport aux valeurs mesurées à J0. Valeurs moyennes de tous les sujets (n = 21).

La **figure 6** décrit les scores cliniques obtenues pour les sujets traités à J14 et J28, comparés à J0. Valeurs moyennes de tous les sujets (n = 21). La comparaison statistique avec J0 (*: p <0,05; N.S .: non significatif) est également montrée.

### Exemples

### A. Résultats biochimiques sur cellules isolées

Ces travaux ont majoritairement été réalisés sur une lignée cellulaire les 3T3-L1, régulièrement utilisée pour les travaux *in vitro* sur les adipocytes. Ces cellules, fibroblastiques, ont la capacité de se différencier en adipocytes matures en réponse à un cocktail de différenciation bien précis.

### A. I. Matériel et Méthodes

### Culture cellulaire

### 1) Entretien de la lignée 3T3-L1

Les cellules ont été cultivées dans un milieu DMEM (Dulbecco's Modified Eagle Medium) complet.

Pour les expériences, les cellules sont trypsinisées puis ensemencées dans les plaques multipuits (MW6 ou MW96). A confluence, le protocole de différenciation peut débuter.

### 2) Protocole de différenciation des 3T3-L1

Le protocole de différenciation se déroule en deux étapes : une phase d'induction (2 jours) suivie d'une étape d'accumulation (2 à 5 jours).
- Milieu d'induction : Il s'agit du milieu d'entretien des 3T3-L1 supplémenté de 1µM d'Insuline ; 1.25µM de Dexaméthasone et 250µM d' IBMX (3-isobutyl-1-methylxanthine)
- Cocktail d'accumulation : Ce milieu est composé du milieu d'entretien des 3T3-L1 supplémenté de 1µM d'Insuline ;

### 3) Entretien des Pré-adipocytes humains (PrAH)

Les cellules sont cultivées dans un milieu DMEM (Dulbecco's Modified Eagle Medium) complet.

Pour les expériences, les cellules sont trypsinisées puis ensemencées dans les plaques multipuits (MW6) :
A confluence, le protocole de différenciation peut débuter.

### 4) Protocole de différenciation des PrAH

Comme pour les 3T3-L1, le protocole de différenciation des PrAH se déroule en deux étapes : les cellules sont exposées à milieu d'induction puis à un milieu d'accumulation, ces deux phases durant 10 jours chacune.

Le milieu d'induction est composé du milieu d'entretien supplémenté de l'insuline à 1µM, de l'IBMX à 500µM, de la dexaméthasone à 1µM ainsi que de l'indométhacine à 200µM. Le milieu d'accumulation est quant à lui composé du milieu d'entretien auquel on ajoute de l'insuline à 1µM.

### Quantification des sphingolipides intracellulaires

Les lipides ont été extraits et les échantillons préparés pour la chromatographie liquide couplée à la spectrométrie de masse (cf. Bligh E.G. et al, 1959).

### 1) Dosage des céramides totaux

Les taux de céramides dans les extraits de lipides cellulaires sont mesurés par la méthode enzymatique de la DAG kinase. Le principe de ce dosage est de phosphoryler les céramides avec de l'ATP radiomarqué grâce à l'action de cette enzyme, puis de quantifier les radiations obtenues pour chaque échantillons, permettant ainsi de doser la quantité totale de céramides présents initialement dans les échantillons. Les résultats sont exprimés en fmol de céramides et normalisés par rapport à la quantité de phospholipides totaux dans les échantillons, exprimée en nmol.

### 2) Mesure des phospholipides totaux pour la normalisation de la quantification des céramides totaux

La quantité de phospholipides totaux contenue dans les extraits cellulaires est dosée afin de normaliser les céramides présents dans chaque échantillon. La quantité de phospholipides totaux est mesurée par spectrophotométrie : l'absorbance est mesurée à 660 nm, puis calculée grâce à une gamme de sodium phosphate dibasique (Sigma-Aldrich) permettant de déterminer la concentration en phosphates dans chaque échantillon.

### Quantification des ARN messagers par RT-qPCR

### 1) Extraction des ARNm

L'extraction des ARNm à partir de ces échantillons est réalisée avec un kit RNeasy^{®} Mini Kit, commercialisé par QIAGEN^{®}.

### 2) Rétrotranscription des ARNm et RT-PCR quantitatives

La rétrotranscription des ARN extraits des échantillons en ADNc est réalisée selon le protocole suivant : les ARN sont incubés à 50°C pendant 30 minutes afin de réaliser la rétro-transcription des ARN en présence de la réversetranscriptase M-MLV ; puis à 95°C pendant 15 minutes afin d'inhiber l'activité de la rétrotranscriptase, de dégrader les amorces d'ARN et d'activer la polymérase.

La PCR quantitative est réalisée dans Le LightCylcer 1.5 (Roche) grâce au kit FastStart DNA Master plus SYBR Green I (Roche).

### Marquages des gouttelettes lipidiques à l'Huile Rouge

Afin de visualiser les gouttelettes lipidiques contenues dans les adipocytes matures et donc de quantifier le niveau de différenciation des cellules, les lipides sont marqués avec de l'huile rouge. L'huile rouge est un colorant liposoluble qui colore les lipides neutres et donc les triglycérides stockés dans les gouttelettes.

### Tests statistiques

Les expériences ont été répétées à quatre reprises (n = 4). Les analyses statistiques ont été réalisées grâce au test de Student, avec le logiciel Excel.

### A. II. Résultats

### Analyse du profil lipidomique des 3T3-L1 au cours de la différenciation

Une analyse du profile lipidomique des 3T3-L1 a été réalisée par HPLC à différents stades de culture et de différentiation des 3T3-L1 :

Au J0 : A confluence, juste avant la différentiation (= Pre-adipocytes (PreAd))
- Au J2, qui correspond au stade entre le cocktail d'induction et le cocktail d'accumulation (AdJ2)
- Au J4, qui correspond au premier jour après application du protocole de différenciation complet (2 jours d'induction + 2 jours d'accumulation) (AdJ4) ;
- Au J7, qui correspond à des cellules exposées 5 jours au cocktail d'accumulation et qui représentent donc des adipocytes matures, ayant accumulé une grande quantité de lipides (AdJ7).

Le but de cette expérience étant de visualiser le flux des sphingolipides au cours de la différenciation, les quantités de sphinganine ont été évaluées, car elles sont précurseur des céramides dans la voie de synthèse de novo, des céramides mais aussi des sphingolipides plus complexes tels que la sphingomyéline et les glucosylcéramides, constituants l'étape suivant la formation des céramides.

Cette expérience a permis de constater qu'au cours de la différenciation, la quantité de sphinganine (Sa) augmente significativement (Figure 1A). Les Sa étant des précurseurs des céramides formés au cours de la voie de synthèse *de novo,* cela suggère que la synthèse de céramides est accélérée pendant l'adipogenèse.

Cependant, la quantité totale de céramides augmente transitoirement à un stade précoce de la différenciation (AdJ2), précédent la phase d'accumulation, puis revient au niveau basal dans les adipocytes matures (AdJ7) (Figure 1B). Une hypothèse pour expliquer ces différences, serait que les céramides, produits en grande quantité sans toutefois avoir le temps de s'accumuler, sont immédiatement utilisés pour former des sphingolipides complexes.

L'étape suivant la formation des céramides est le transfert de ces derniers dans l'appareil de Golgi soit par une voie vésiculaire, pour former des glucosylcéramides (GlcCer), soit grâce au transporteur de céramides CERT, pour former des sphingomyélines (SM) (Giussani et al., 2008; Hanada, 2003; van Meer et Sprong, 2004; Yamaji et al., 2008).

Les résultats de l'analyse lipidomique de nos échantillons montrent que si les quantités de GlcCer semblent suivre le même profil que les Cer - avec une augmentation transitoire lors de la phase précoce de différenciation, puis un retour à un niveau basal (Figure 1D), il n'en est pas de même pour les SM dont la quantité augmente significativement et de façon temps dépendant au cours de l'adipogenèse (Figure 1C). L'ensemble de ces résultats ont ainsi permis de conclure que la voie de synthèse de novo est bien activée au cours de l'adipogenèse, et que ceci est associé à la formation en grande quantité de SM.

L'expression de la sphingomyéline synthase 1 (SMS1), enzyme responsable de la synthèse des SM dans l'appareil de Golgi, a été quantifée par RT-qPCR au cours de la différenciation adipocytaire, mais celle-ci n'est pas induite au cours de l'adipogenèse (Figure 1E). En revanche, l'expression du transporteur CERT, qui contribue à la synthèse de la sphingomyéline en transportant les céramides du RE vers l'appareil de Golgi (Giussani et al., 2008; Hanada, 2003; van Meer and Sprong, 2004; Yamaji et al., 2008), est très fortement induite au cours de la différenciation adipocytaire (Figure 1F).

Ces résultats mettent en valeur un potentiel rôle du transporteur CERT dans l'adipogenèse.

Les effets de son inhibition - et donc du blocage du transport des Cer vers l'appareil de Golgi - ont été étudiés sur la capacité de différenciation des pré-adipocytes.

### Validation de l'inhibition de CERT par la Gédunine

Des inhibiteurs de CERT existent et sont bien connus, notamment le HPA-12 (N-(3-Hydroxy 1-hydroxymethyl-3-phenylpropyl)dodecanamide) dont l'activité inhibitrice a été démontrée en 2001 par l'équipe de Hanada (Yasuda et al., 2001).

Un autre inhibiteur connu, existant de manière naturelle dans des plantes, a également été testé. Il s'agit de la Gédunine (GED), un limonoïde présent dans certaines plantes et notamment dans le Margousier (Hullin-Matsuda et al., 2012).

Dans un premier temps, il a été confirmé que l'inhibition de CERT par la Gédunine impliquait une interaction directe entre la Gédunine et la protéine CERT. Pour ce faire, une étude in silico a été réalisée. Classiquement, les inhibiteurs de CERT, de la famille des HPA, interagissent avec le domaine START de la protéine, inhibant leur liaison avec les céramides (Yasuda et al., 2001). Pour savoir si la GED interagit avec CERT de la même manière, les potentielles interactions entre le domaine START de la protéine et la molécule GED ont été déterminées grâce au logiciel AutoDockTools^{®}, qui permet de calculer l'énergie nécessaire à chaque pose ainsi que la constante d'inhibition (Ki) et les distances (en Ångström, Å) entre les résidus de la protéine et la molécule (la gédunine).

Cette analyse a permis de constater qu'une interaction directe entre la GED et le domaine START de CERT est possible ; et que celle-ci, d'un point de vue énergétique est tout à fait envisageable puisque l'énergie demandée (exprimée en kcal/mol) est négative, de l'ordre de -11,47 kcal/mol. De plus, le Ki résultant de cette pose, d'une valeur de 3,93 nM, est très bas, suggérant que le potentiel d'inhibition de la GED sur CERT serait robuste.

| Domaine START (3h3r) | Energie (kcal/mol) | Ki (nM) |
|---|---|---|
| **HPA-14** | -7,99 | 869 |
| **Gédunine** | -11,47 | 3,93 |

Enfin, les distances entre les résidus de la protéine CERT et la GED, de l'ordre de 3 Â en moyenne, sont également faibles. Par ailleurs, il est intéressant de noter que cette approche a mis en évidence une interaction possible entre les résidus TYR 553 et GLN467 de la protéine CERT avec la Gédunine. Or, une étude publiée en 2010 a montré par co-cristallisation que le ligand HPA 15, inhibiteur connu de CERT, interagit avec la protéine au niveau de ces mêmes résidus, renforçant l'idée qu'une interaction directe entre CERT et la Gédunine est possible (Kudo et al., 2010). Cette analyse *in silico* suggère que la GED est capable de se fixer sur le domaine START de CERT et ainsi qu'une inhibition directe est tout à fait possible.

### Détermination de la concentration de gédunine nécessaire et suffisante à l'inhibition de CERT

Après avoir observé in silico que la gédunine est un bon inhibiteur de la protéine CERT, ses effets sur la capacité de différenciation des 3T3-L1 ont été testés. Un test de dose-réponse a été réalisé sur des 3T3-L1 à l'état basal, permettant d'une part d'observer la potentielle toxicité de la molécule; et d'autre part, d'évaluer le degré d'inhibition de CERT par un dosage des céramides totaux présents dans les échantillons, témoins de la capacité du transporteur à transférer les céramides du RE vers le Golgi pour la synthèse des SM.

Le dosage des céramides totaux montre que les céramides commencent à s'accumuler à partir d'une dose de GED de 10µM sans aucune toxicité (donnée non montrée). Au vu de ce premier résultat, la concentration de GED de 10µM a été choisie pour le reste de ce travail.

### Effet d'un traitement à la Gédunine sur les quantités de sphingolipides au cours de la différenciation

Afin de déterminer si la GED est capable d'inhiber le transport des céramides du RE vers le Golgi au cours du processus de différenciation des 3T3-L1, ces cellules ont été mises en contact de la GED au cours des deux phases du protocole (Schéma 3). Puis les céramides totaux et les sphingomyélines ont été quantifiés (figure 2A).

Les résultats du dosage des céramides totaux dans les échantillons traités ou non à la GED donnent deux informations. Premièrement, une accumulation des céramides significative par rapport aux échantillons contrôles est observée aussi bien dans les AdJ2 traités dans la phase d'induction que dans les AdJ7 traités durant l'étape d'accumulation, témoignant d'un blocage de l'action de transporteur de CERT et donc de l'action inhibitrice de la GED. Par ailleurs, les échantillons qui ont été traités à la GED durant l'étape d'induction puis qui ont été cultivés dans un milieu d'accumulation contrôle, sans GED, ne présentent pas d'accumulation des céramides, suggérant que l'activité inhibitrice de la GED est réversible dans le temps (Figure 2B). L'action inhibitrice de la GED sur l'enzyme CERT a donc été validée.

Le dosage des SM a permis d'observer qu'à un niveau précoce de la différenciation, juste avant l'étape d'accumulation, il y a une légère diminution de la quantité de SM, toutefois non significative tandis qu'à une phase plus tardive, après 5 jours d'accumulation, les traitements à la GED, aussi bien durant la première que durant la deuxième étape du protocole, permettent de diminuer significativement l'accumulation de SM (Figure 2C). L'accumulation de céramides et la diminution parallèle de SM en réponse à un traitement à la GED valident ainsi le protocole d'inhibition de CERT avec une concentration de 10µM de gédunine.

### Effet de la Gédunine sur la capacité de différenciation des 3T3-L1

L'inhibition de CERT, et donc de la synthèse de SM, par le traitement à la GED ayant été validée, la question a donc ensuite été de savoir si cela avait un effet sur la capacité de différenciation des cellules. Le même protocole de différenciation a donc été appliqué, associé ou non à un traitement à la Ged (figure 2A), et les niveaux d'expression de gènes impliqués d'une part dans l'adipogenèse et d'autre part dans la fonction adipocytaire ont été mesurés. Par ailleurs, les analyses transcriptomiques ont été combinées avec une analyse phénotypique des échantillons : un marquage à l'huile rouge, qui, en marquant les lipides, permet de visualiser les gouttelettes lipidiques dans les cellules et donc de savoir si celles-ci sont différenciées et fonctionnelles.

Les niveaux d'expression des gènes Pref1, comme marqueur pré-adipocytaire, ainsi que de PPARγ, C/EBPα et C/EBPδ qui sont des FT clés dans l'adipogenèse, ont été mesurés.

Au cours du protocole de différenciation des 3T3-L1, l'expression de Pref1 (Figure 3A) est diminuée comme attendu dans les échantillons contrôles (CTRL), Pref1 n'étant exprimé que dans les pré-adipocytes. En revanche, l'analyse montre que dans les échantillons traités à la GED durant la phase d'induction (appelés « GED1 »), l'expression de Pref1 diminue de la même manière que chez les CTRL au stade AdJ2, mais n'est plus réprimé au stade AdJ7, suggérant que les cellules ne perdent plus complètement leur phénotype pré-adipocytaire. Cependant, dans les cellules traitées avec la GED durant l'étape d'accumulation (appelés « GED2 »), l'expression de Pref1 est identique à celle des CTRL au stade AdJ7. Ce résultat est attendu car l'expression de Pref1 est perdue précocement au cours de l'adipogenèse et que dans ces dernières conditions (GED2), les cellules ont été soumises à une première phase d'induction contrôle, sans traitement.

Dans les échantillons CTRL, l'expression de PPARγ est bien induite au cours de la différenciation (Figure 3A), comme cela était attendu, PPARγ étant un facteur de transcription dont l'expression est nécessaire et suffisante à l'adipogenèse. De manière intéressante, ces résultats ont montré que le traitement des cellules avec la GED au cours de la phase d'induction entraîne une diminution de cette induction de l'expression de PPARγ au stade AdJ2, soit juste après le traitement, mais également au stade AdJ7 dans les échantillons qui ont ensuite été soumis à un milieu d'accumulation classique (sans GED). De plus, dans les échantillons traités à la GED durant la phase d'accumulation, une diminution drastique de l'induction de l'expression de PPARγ est observée. Il en est de même avec l'expression de C/EBPα, dont l'induction au cours de l'adipogenèse observée dans les CTRL est diminuée dans les échantillons GED1 et GED2 au stade AdJ7. En effet, son expression est induite par PPARγ et est donc plus tardive (Rieusset et al., 2002), elle n'est donc pas observée au stade AdJ2 dans le protocole utilisé ici (Figure 4A).

Enfin, le facteur de transcription C/EBPδ est normalement induit de façon transitoire et précocement lors de la différenciation puis son expression diminue au cours des étapes plus tardives de l'adipogenèse. C'est bien ce qui est observé dans les échantillons CTRL (Figure 3A). De manière intéressante, si le traitement à la GED semble diminuer, toutefois non-significativement, l'induction de l'expression de C/EBPδ au stade AdJ2, il a un effet inverse aux stades plus tardifs. En effet, il a été observé qu'aussi bien dans les cellules GED1 que GED2, au stade AdJ7, le niveau d'expression de C/EBPδ est significativement augmenté par rapport aux échantillons CTRL, suggérant que le traitement à la GED perturbe la régulation de l'expression de ce facteur de transcription précoce.

Ainsi, l'ensemble de ces résultats démontre que le traitement à la Gédunine, que ce soit au cours de la phase d'induction ou d'accumulation, bloque le processus d'adipogenèse des 3T3-L1 comme en témoignent les altérations des profils d'expression de facteurs de transcription nécessaires à la différenciation adipocytaire.

L'effet de la GED sur l'expression de gènes codant des protéines nécessaires à la fonction adipocytaire, telles que CD36, FAS, LPL et sur l'adiponectine, a également été mesuré. Les expressions de ces 4 gènes sont fortement induites au cours de l'adipogenèse dans les cellules CTRL, comme attendu (Figures 3B et C). De façon intéressante, le traitement à la GED, aussi bien durant la phase d'induction que d'accumulation, diminue l'induction de l'expression de ces 4 gènes aux stades AdJ2 et AdJ7, avec toutefois un effet plus fort du traitement durant l'étape d'accumulation : au niveau de l'induction de l'expression de ces 4 gènes une diminution drastique est observée dans les échantillons AdJ7 GED2 (Figures 3B et C).

Le marquage des lipides neutres par l'huile rouge, réalisés sur des cellules au stade tardif de l'accumulation, à AdJ7, montrent que les cellules CTRL ont bien été capables de stocker des lipides sous forme de gouttelettes lipidiques, marquées en rouge. En revanche, les cellules traitées à la GED au cours de la phase d'induction (GED1) ou d'accumulation (GED2) ne sont plus marquées, révélant que celles-ci ne sont plus capables de stocker des lipides et ne constituent donc pas des adipocytes matures (Figure 3D).

L'ensemble de ces résultats ont permis de constater que le traitement à la Gédunine pendant la phase d'induction impacte négativement la capacité de différenciation des 3T3-L1. Toutefois, il semble que le traitement GED durant l'étape d'accumulation ait un effet plus fort sur l'inhibition de l'adipogenèse, entraînant des diminutions drastiques de l'induction de l'expression de gènes majeurs pour la fonction adipocytaire. Ainsi, ces résultats suggèrent que le blocage de la synthèse des SM par l'inhibition du transport des céramides du RE vers l'appareil de Golgi impacte négativement la capacité de différenciation des pré-adipocytes, mettant en évidence un nouveau rôle du métabolisme des céramides dans ce processus.

### Effets de la Gédunine sur la capacité de différenciation de pré-adipocytes humains in vitro

Le modèle cellulaire 3T3-L1 utilisé a permis de montrer que l'inhibition de la protéine CERT, et ainsi que le transport des céramides à l'origine de la synthèse de SM, au cours de la phase tardive du processus de différenciation est capable de bloquer l'adipogenèse. Dans le but de pouvoir appliquer les résultats de ce projet à la création d'une nouvelle crème amincissante, il a ensuite fallu montrer que les effets observés dans la lignée cellulaire 3T3-L1 étaient transposables à un modèle d'adipocytes humains.

Les expériences ont donc été reproduites sur des pré-adipocytes humains normaux. Dans un premier temps, le niveau d'expression du transporteur CERT a été mesuré par RT-qPCR. Celui-ci est également induit dans les cellules humaines au cours de la différenciation, de la même manière que dans les 3T3-L1 (résultat non montré).

Dans un premier temps, les cellules ont donc été traitées avec de la Gédunine durant la phase d'induction. Le niveau d'expression de gènes impliqués dans l'adipogenèse et dans la fonction adipocytaire a été évalué. Dans un deuxième temps, les cellules ont été colorées avec de l'Huile Rouge afin de visualiser leur capacité à stocker des lipides au sein de leurs gouttelettes lipidiques.

L'analyse par RT-qPCR des niveaux d'expression de PPARγ, Pref1, AdipoQ, FAS et LPL a permis d'observer que chez les cellules CTRL après induction de la différenciation (nommées AH), l'expression de PPARγ est fortement induite, atteignant un niveau 22 fois plus élevé que dans les PrAH ; et que cela est corrélé avec une diminution de l'expression de Pref1, qui est égal à 40% du niveau d'expression dans les PrAH. De plus, les expressions des gènes codants pour l'adiponectine, FAS et LPL sont également fortement induites au stade AH, validant le protocole de différenciation des pré-adipocytes humains (Figures 4A et 7B).

De plus, il a été observé que dans les cellules AH traitées avec de la Gédunine (nommées GED), l'induction de l'expression de PPARγ est fortement diminuée par rapport au CTRL et cela est couplé à la diminution des expressions de AdipoQ, FAS et LPL. Ceci permet de conclure que le traitement à la Gédunine est capable de bloquer l'adipogenèse dans les pré-adipocytes humains (Figures 4A et 7B). Cependant, aucune différence significative n'a été relevée dans l'expression du marqueur pré-adipocytaire Pref1 entre les cellules CTRL et GED, suggérant que le traitement à la gédunine n'influence pas l'expression de ce gène.

### B. Caractérisation de l'effet anti-adipogénique de la limonine

### B.1. Matériel et Méthodes

Brièvement : Des cellules 3T3-L1 ont été ensemencés dans leur milieu d'entretien. A confluence, les cellules ont été mises au contact des produits à tester dans un milieu permettant la différentiation des pré-adipocytes en adipocytes via la présence d'un cocktail d'induction. A l'issue du contact, les lipides neutres stockés dans les gouttelettes lipidiques des adipocytes ont été marqués par la coloration Huile Rouge O. Les lipides neutres ont été extraits (extraction à l'isopropanol) et dosés par spectrophotométrie. Les densités optiques obtenues sont proportionnelles à la quantité de lipides.

La limonine testée est commercialisée par Sigma Aldrich.

L'extrait de mandarine testé est commercialisé par SEDERMA (SE28226). Il contient 99% de triglycéride caprylique et 1% d'extrait de fruit de Citrus nobilis.

### B.2. Résultats

### Etude de l'activité de la limonine sur les cellules adipocytes

| Concentrations en Limonine | Moyenne ± écart type (Densité optique à 490nm) | % variation versus contrôle |
|---|---|---|
| 0 | 0,545 ± 0,060 | Référence |
| 10µM | 0,492 ± 0,061 | -10% |
| 2OµM | 0,455 ± 0,037 | -16,5% |

Dans les conditions de la présente étude :
- Aucune cytotoxicité n'a été observée pour les concentrations testées.
- Une baisse dose dépendante de la quantité de lipides a été observée en présence de la Limonine par rapport au contrôle. Ceci traduit un ralentissement/inhibition de la différentiation adipocytaire en présence de la Limonine.

### Etude de l'activité d'un extrait de mandarine titré en limonine

| Concentrations de l'extrait de Mandarine (Réf PROD-19-009) | Moyenne ± écart type (Densité optique à 490nm) | % variation versus contrôle |
|---|---|---|
| 0 | 0,714 ± 0,092 | Référence |
| 8 µg/mL | 0,599 ± 0,024 | -16%; p<O.05 |
| 15 µg/mL | 0,549 ± 0,017 | -23% ; p<0.01 |
| 61 µg/mL | 0,491 ± 0,018 | -31% ; p<0,01 |

Dans cette expérience,
- Aucune cytotoxicité n'a été observée pour les concentrations testées.
- Une baisse dose dépendante de la quantité de lipides a été observée en présence de la Limonine par rapport au contrôle. Ceci traduit un ralentissement/inhibition de la différentiation adipocytaire en présence de la Limonine.

### C. Confirmation de l'effet amincissant du produit de l'invention après application sur la peau

### C.1. Matériel et Méthodes

Ce projet est une étude monocentrique contrôlée chez des sujets sains. Cette étude a été réalisée conformément aux bonnes pratiques cliniques établies pour le développement d'un projet de recherche impliquant des sujets humains, résumées par protocole (MD.122 / 02 Protocol PT.08.01 / final02).

### Sujets testés

21 sujets ont été inclus dans le test. Les caractéristiques de ces sujets sont les suivantes :
- Âge: 31-58 ans,
- Sexe: femme,
- Phototype (Fitzpatrick): Il à III,
- Tous types de peaux dont 52% de peaux sensibles,
- Femmes avec un IMC compris entre 21,2 et 25,9, présentant de la cellulite sur les cuisses (visible à l'œil nu), avec un aspect peau d'orange, des rondeurs localisée (cuisses, hanches, ventre) et un manque de fermeté / tonicité sur ces zones,
- Ayant un poids stable.

### Produit testé

### SOIN CORPS : BODY CARE SC028, FORMULA A056, BATCH 192810

| **Produit** | **Formule** | **Lot** | **Application** | **Forme galénique** | **Quantité administrée pendant l'étude / nombre** |
|---|---|---|---|---|---|
| SOIN CORPS : SC028 | A056 | 192810 | Deux fois par jour | EMULSION | 25 X 150g |

### Protocole de l'étude

| **Produit** | **Zones** | **Utilisation** | **Fréquence** | **Quantité** |
|---|---|---|---|---|
| SOIN CORPS: SC028 | Corps, en insistant sur les cuisses, les hanches, ventre, fesses et bras | Dans les conditions normales d'emploi | À partir de J0 : le matin au centre de test puis le soir à domicile jusqu'à J27±2 (4 semaines) Deux fois par jour. | Autant que nécessaire |

Les sujets ont été interrogés pendant et à la fin de l'étude sur la façon dont ils ont utilisé le produit testé. L'enquêteur a évalué l'importance des écarts possibles par rapport aux conditions expérimentales requises au début de l'étude. L'enquêteur a vérifié le respect des contraintes.

Tous les écarts par rapport au protocole ont été analysés et l'investigateur a évalué leur effet sur la validité des résultats. Toutes les contraintes de l'étude, définies dans le protocole, ont été respectées par les sujets.

Un contrôle de la consommation du produit a été réalisé à la fin de l'étude. Le ou les échantillons fournis à chaque sujet avaient un poids au début de l'étude et à la fin après la dernière application. La consommation moyenne par application a été de 2,96g ± 0,36g.

Le produit « SOIN CORPS SC028 » est un produit selon l'invention. Il contient *a minima* 1% d'extrait de *Citrus nobilis,* comme décrit dans le tableau ci-dessous.

| **N° CAS** | **INCI Ingrédients INCI** | **Fonction** | **% p/p** |
|---|---|---|---|
| 7732-18-5 | EAU | *SOLVANT* | qsp 100 |
| 64-17-5 | ALCOOL DENAT. | *SOLVANT* | 15-20 |
| 5343-92-0 | PENTYLÈNE GLYCOL | *SOLVANT* | 2-5 |
| 107-88-0 | BUTYLENE GLYCOL | *SOLVANT* | 2-5 |
| 194043-92-0 | Beurre de karité BUTYROSPERMUM PARKII | *Agent pour la peau* | 1-3 |
| 58-08-2 | CAFEINE | *Agent pour la peau* | 1-3 |
| 68201-46-7 | COCOATE DE GLYCÉRYLE PEG-7 | *Emulsifiant* | 1-3 |
| 73398-61-5 | TRIGLYCÉRIDE CAPRYLIQUE / CAPRIQUE | *Agent pour la peau* | 1-3 |
| 111286-86-3 | Copolymère ACRYLATE D'HYDROXYÉTHYLE / TAURATE D'ACRYLOYLDIMETHYLE DE SODIUM | *Agent de viscosité aqueux* | 1-3 |
| 352458-32-3 | Huile de graine de CARAPA GUAIANENSIS | *Agent pour la peau* | 1-3 |
| 100843-69-4 | SACCHARIDE ISOMERATE | *Agent pour la peau* | 0,2-1 |
| 7695-91-2 | ACETATE DE TOCOPHEROL | *ANTIOXIDANT* | 0,2-1 |
| 68956-68-3 | Extrait de graine de SCHINUS TEREBINTHIFOLIA | *Agent pour la peau* | 0,05-0,2 |
| 91770-92-2 | Extrait de ZINGIBER ZERUMBET | *Agent pour la peau* | 0,05-0,2 |
| 84929-38-4 | Extrait de fruit de CITRUS NOBILIS (MANDARIN ORANGE) | *Agent pour la peau* | 0,01-0,03 |
| 8000-28-0 | Huile essentielle de LAVANDULA ANGUSTIFOLIA (LAVENDE) | *Ingrédients de fragrance* | 0,05-0,2 |
| 84837-14-9 (EU) | Huile essentielle de fleur de THYMUS MASTICHINA | *Ingrédients de fragrance* | 0,05-0,2 |
| 77-53-2 | CEDROL | *Ingrédients de fragrance* | 0,05-0,1 |
| 8000-25-7 | Huile essentielle de feuille de ROSMARINUS OFFICINALIS (ROMARIN) | *Ingrédients de fragrance* | 0,05-0,1 |
| 8022-56-8 | Huile essentielle de SALVIA OFFICINALIS (SAUGE) | *Ingrédients de fragrance* | 0,05-0,1 |
| 29806-73-3 | PALMITATE D'ÉTHYLHEXYLE | *Agent pour la peau* | 0,5-2 |
| 5333-42-6 | OCTYLDODECANOL | *Agent pour la peau* | 0,5-2 |
| 504-63-2 | PROPANEDIOL | *SOLVANT* | 0,2-1 |
| 423772-95-6 | OCTYLDODECYL XYLOSIDE | *Surfactant* | 0,2-1 |
| 439612-67-6 | PEG-30 DIPOLYHYDROXYSTEARATE | *Surfactant* | 0,2-1 |
| 9005-67-8 | POLYSORBATE 60 | *Surfactant* | 0,05-0,2 |
| 54392-26-6 71902-01-7 | ISOSTÉARATE DE SORBITAN | *Surfactant* | 0,05-0,2 |
| 5949-29-1 77-92-9 | ACIDE CITRIQUE | *AJUSTEUR DE PH* | 0,03-0,15 |
| 7558-79-4 | PHOSPHATE DE DISODIUM | *AJUSTEUR DE PH* | 0,02-0,10 |
| 11138-66-2 | GOMME DE XANTHANE | *Agent de viscosité aqueux* | 0,02-0,1 |
| 13472-35-0 | PHOSPHATE DE SODIUM | *Tampon* | 0,01-0,05 |
| 26402-26-6 | CAPRYLATE DE GLYCERYLE | *Surfactant* | 0,01-0,05 |
| 68-04-2 | CITRATE DE SODIUM | *AJUSTEUR DE PH* | 0,005-0,01 |
| 1310-73-2 | HYDROXYDE DE SODIUM | *AJUSTEUR DE PH* | 0,005-0,01 |
| 10191-41-0 | TOCOPHEROL | *ANTIOXIDANT* | 0,001-0,002 |
| | TOTAL | | 100 |

### Questionnaires

Les sujets se sont auto-évalués pendant (à J0 et à J14) et à la fin (J28) de l'étude. Ce questionnaire regroupe les éléments concernant l'efficacité du produit :
- Fermeté de la peau (entre 0 : non ferme et 9 : très ferme)
- Aspect galbé de la cuisse (entre 0 : très rond et sans forme et 9 : bien galbé et très défini)
- Aspect lisse de la peau (entre 0 : pas lisse et 9 : très lisse)
- Présence de rondeurs (entre 0 : peu de rondeurs et 9 : beaucoup de rondeurs)
- Inconfort au pincement sur une zone avec cellulite (entre 0 : pas d'inconfort, et 9 : très inconfortable)
- Aspect de la cellulite au pincement (entre 0 : pas lisse et 9 : très lisse)
- Aspect visuel de la cellulite (entre 0 : pas détectable et 4 : « effet matelassé »)

Tous les sujets inclus dans l'étude ont été pris en compte pour évaluer l'efficacité de la procédure.

Pour chaque élément, les sujets répondent selon une échelle de notation définie et les résultats sont exprimés en pourcentage de variation.

### Quantification de l'effet sur la cellulite

### 1/ Par topographie de la peau

Des images en 3D de la topographie de la peau ont été obtenues par une caméra stéréo combinée à un système de projection de franges. Des franges standardisées sont projetées sur la peau et détectée par les doubles caméras du système optique. L'effet 3D est calculé par la déformation des franges qui représente qualitativement et quantitativement le relief de peau et par la stéréo-combinaison de l'image projetée par les deux caméras.

Un système de mesure in-vivo de la peau a été utilisé (AEVA-HE, Eotech, France). Le logiciel AEVA est une plate-forme universelle pour l'acquisition, l'analyse et l'évaluation des données 3D de la peau.

Les mesures ont été effectuées sur la cuisse (droite ou gauche selon la randomisation), à J0, J14, ou J28.

### 2/ Par échographie

Les images échographiques ont été générées avec une sonde de 20 MHz. Les tissus échogènes sont détectés et une image échographique en mode B est générée. Les échographes détectent correctement les structures du derme. L'épaisseur de la graisse sous-cutanée peut être obtenue en observant la jonction derme-hypoderme et le fascia musculaire. Un système à ultrasons Dermascan C (Cortex Technology, Danemark) avec une sonde à ultrasons spéciale modifiée de 20 MHz a été utilisé. Les mesures ont été effectuées sur la cuisse gauche ou droite (selon la randomisation) à J0, J14, ou J28.

À chaque temps de l'étude, l'épaisseur du tissu adipeux a été calculé. Les calculs ont été effectués sur l'image échographique en mode B de chaque zone.

Tous les sujets inclus dans l'étude ont été pris en compte pour évaluer l'efficacité du produit à tester tant qu'ils ont été soumis à tous les examens, aux moments définis.

### 3/ Mesure de la fermeté et de l'élasticité de la peau

L'évaluation des propriétés biomécaniques de la peau a été réalisée par un cutomètre dual MPA 580 à l'aide d'une sonde de 8 mm. Ce système est utilisé pour mesurer l'élasticité des couches supérieures de la peau en utilisant une pression négative qui déforme la peau mécaniquement. Le principe de mesure est basé sur la méthode d'aspiration. Une pression négative est créée dans l'appareil (450 mbar) et la peau est entraînée dans l'ouverture de la sonde (3s) et après un temps défini relâchée à nouveau (3s). À l'intérieur de la sonde, la profondeur de pénétration est déterminée par un système de mesure optique sans contact qui se compose d'une source de lumière et d'un récepteur de lumière, ainsi que de deux prismes se faisant face, qui projettent la lumière de l'émetteur au récepteur. L'intensité lumineuse change en fonction de la profondeur de pénétration de la peau. La résistance de la peau à la pression négative (fermeté) et sa capacité à revenir dans sa position d'origine (élasticité) sont affichées sous forme de courbes (profondeur de pénétration en mm / temps) en temps réel pendant la mesure. Ce principe de mesure permet d'obtenir des informations sur les propriétés élastiques et mécaniques de la surface cutanée. Les mesures sont effectuées avec un cutomètre dual MPA 580 (Courage & Khazaka) équipé d'une sonde de 8 mm de diamètre et qui génère une pression négative de 450 mbars permettant d'étirer verticalement les couches supérieures de la peau. La sonde est située sur un anneau adhésif pour immobiliser parfaitement le site cutané. Les mesures ont été effectuées sur la cuisse gauche ou droite (selon la randomisation) à J0, J14, ou J28.

A chaque temps de l'étude, les propriétés biomécaniques de la peau sont représentées par une courbe de déformation en fonction du temps. À partir de cet enregistrement, les paramètres suivants sont conservés:

R2 (Ua/Uf) (en %): rapport entre la capacité de revenir à la position d'origine et l'amplitude maximale. Il s'agit d'un paramètre d'élasticité standard. Plus la valeur est proche de 1 (100%), plus la peau est élastique.

RO (Uf) (en mm): Point le plus haut de la première courbe. Ce paramètre représente le comportement passif de la peau à la force et est une mesure de fermeté. Un résultat bas est lié à un effet raffermissant plus élevé.

### 4/ Mesures en cm

Le périmètre des cuisses a été obtenu avec un ruban à mesurer souple. L'appareil de mesure était un ruban SECA 201 avec une précision de 1 mm. Toutes les mesures ont été obtenues en mm. Les mesures ont été effectuées sur la cuisse gauche ou droite (selon la randomisation) à J0, J14, ou J28.

### 5/ Evaluation de la cellulite par un score clinique

L'état de la cellulite a été évaluée à chaque point de l'étude par un système d'évaluation universel de la cellulite appelé « échelle de Nurnberger-Muller ». Cette évaluation a été réalisée par un dermatologue.

| **Grade** | **Debout** | **Couché** | **Après pincement** |
|---|---|---|---|
| Stade 0 | absence | absence | absence |
| Stade 1 | absence | absence | Cellulite visible |
| Stade 2 | Cellulite visible | absence | Cellulite visible |
| Stade 3 | Cellulite visible | Cellulite visible | Cellulite visible |

| | | | |
|---|---|---|---|
| Stade 0 : correspond à l'absence de cellulite Stade 1 : la cellulite est visible seulement lorsqu'on pince la peau. Stade 2 : elle est visible lorsqu'on se tient debout, mais pas en position allongée. Stade 3 : elle est visible aussi bien en position debout qu'allongée. | | | |

### 6/ Evaluation par l'échelle photonumérique d'Hexsel

Il est fondamental de recourir à l'utilisation de paramètres objectifs en combinaison avec des échelles photonumériques et la perception des patients comme paramètres d'efficacité dans les essais cliniques contrôlés et randomisés.

L'intensité de chaque élément (A à E) est notée de 0 à 3, ce qui permet de calculer une somme finale de scores variant de 1 à 15. La cellulite est en outre classée comme légère (score total: 1-5), modérée (score total: 6-10) ou sévère (score total: 11-15).

Nombre de dépressions évidentes (A) : 0: aucune dépression / 1: 1 à 4 dépressions/ 2: 5 à 9 dépressions /3 : 10 ou plus de dépressions

Profondeur des dépressions (B) : 0: aucune dépression / 3: Superficielle / 4: Profondeur moyenne / 5: Profonde

Aspect morphologique de la surface cutanée (C) : 0: aucune zone surélevée / 6: "peau d'orange" / 7: "Cottage cheese" / 8: "effet Matelassé"

Degré de laxité et de relâchement cutané (D) : 0: Absence de relâchement, de flaccidité ou d'affaissement de la peau / 9: Léger / 10: Modéré / 11: Sévère

Classement de Nurnberger et Muller (E) : 0: non détectable / 4: Léger - présence de capitons lorsqu'une pression est appliquée / 5: Modéré - Les capitons sont visibles en position debout mais pas en position couchée / 6: Grave - capitons en positions debout et couchée

Le score est calculé de la façon suivante: CSS (cellulite severity scale) = A + B+ C+ D + E

Les mesures ont été effectuées sur la cuisse gauche ou droite (selon la randomisation) à J0, J14, ou J28.

### Tests statistiques

Tous les sujets inclus dans l'étude ont été pris en compte pour évaluer l'efficacité du produit à tester tant qu'ils ont été soumis à tous les examens, aux temps définis.

Tous les résultats individuels ont été exprimés:
. En valeurs absolues du paramètre pour chaque temps de l'étude,
. En variation du paramètre contre J0 pour chaque temps de l'étude.
. En % de variation des valeurs des paramètres et variation de surface de chaque temps intermédiaire par rapport à J0.

Les données d'efficacité instrumentale sont soumises à un traitement statistique approprié (Student-t ou Wilkoxon Ranks Signs Test pour toutes les comparaisons de données continues entre J0, J14 et J28).

Tous les calculs ont été effectués à l'aide du logiciel SPSS 20 (IBM). Un niveau de significativité de 95% a été adopté. Les données subjectives d'efficacité ont été soumises à un test binomial de traitement statistique adapté.

### C.2. Résultats

### 1/ Concernant l'efficacité clinique du produit :

Le produit SOIN CORPS: BODY CARE SC028, FORMULA A056 a présenté une diminution de 5,81% et 6,53% du paramètre Ra d'ondulation cutanée, sur la zone de la cuisse avec cellulite et une diminution de 6,84% et 3,09% sur la zone de la cuisse sans cellulite, après 14 et 28 jours d'application, respectivement. Ces changements sont statistiquement significatifs.

Les résultats sont visibles sur le tableau 1 :

**Tableau 1 - valeurs de l'ondulation cutanée (Ra et Rz) pendant l'étude, évaluées sur deux zones de la cuisse, une avec cellulite, l'autre sans cellulite.**

| Zone Cuisse aveccellulite | Paramètre | Temp s | n | Moyen ne | déviati on standa rd | Médiane | Minimum | Maximum | p |
|---|---|---|---|---|---|---|---|---|---|
| | Ra | J0 | 21 | 0,111 | 0,062 | 0,097 | 0,050 | 0,278 | |
| | | J14 | 21 | 0,105 | 0,058 | 0,087 | 0,045 | 0,248 | **0,025** |
| | | J28 | 21 | 0,104 | 0,058 | 0,093 | 0,046 | 0,248 | **0,038** |
| | Rz | J0 | 21 | 0,150 | 0,094 | 0,117 | 0,073 | 0,402 | |
| | | J14 | 21 | 0,138 | 0,079 | 0,100 | 0,056 | 0,375 | **0,009** |
| | | J28 | 21 | 0,141 | 0,088 | 0,098 | 0,060 | 0,421 | 0,206 |
| Cuisse sans cellulite | Ra | J0 | 21 | 0,059 | 0,033 | 0,045 | 0,025 | 0,131 | |
| | | J14 | 21 | 0,055 | 0.029 | 0,042 | 0,024 | 0,109 | **0.014** |
| | | J28 | 21 | 0,057 | 0,036 | 0,040 | 0,022 | 0,145 | **0,027** |
| | Rz | J0 | 21 | 0,078 | 0,038 | 0,068 | 0,033 | 0,152 | |
| | | J14 | 21 | 0,071 | 0,035 | 0,061 | 0,033 | 0,149 | **0,005** |
| | | J28 | 21 | 0,074 | 0,038 | 0,066 | 0,034 | 0,147 | 0,053 |

En résumé, après 14 et 28 jours, à la suite de l'application du produit, il y a une diminution significative du paramètre Ra d'ondulation cutanée, dans les deux zones de la cuisse (avec et sans cellulite).

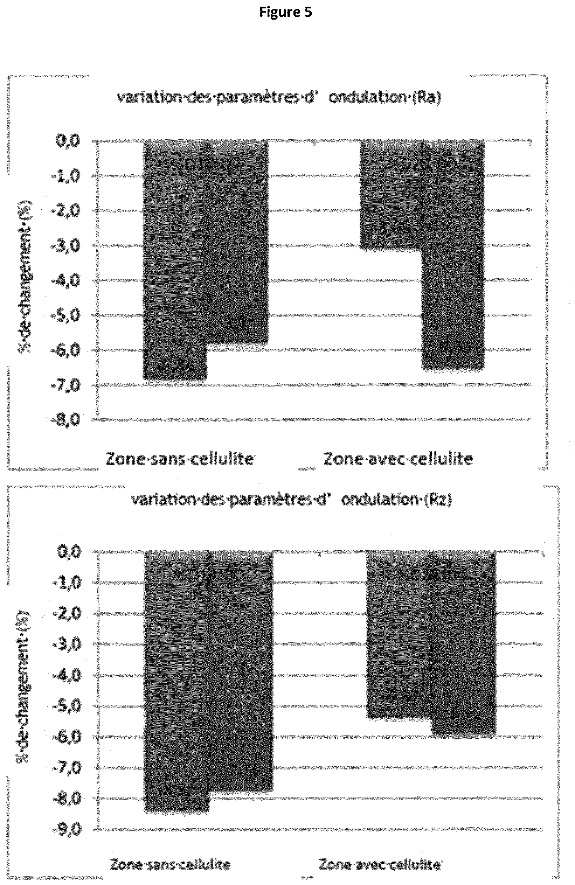

Pour évaluer les vrais changements après 14 et 28 jours, la transformation relative par rapport à J0 a été effectuée. Les résultats sont résumés dans la figure 5.

Les autres paramètres ont été évalués de la même façon (non montré).

Les résultats sont les suivants :

Le produit SOIN CORPS: BODY CARE SC028, FORMULA A056 a présenté une diminution de 9,91% et 9,48% des paramètres de rugosité cutanée Ra et Rz, respectivement, sur la zone de la cuisse avec cellulite, après 28 jours d'application. Ces changements sont statistiquement significatifs.

Le produit SOIN CORPS: BODY CARE SC028, FORMULA A056 a présenté une diminution de 0,41% et 0,66% du volume de la cuisse et une diminution de 0,91% et 1,42% du périmètre de la cuisse, après 14 et 28 jours d'application, respectivement. Ces changements sont statistiquement significatifs après 28 jours d'application.

Le produit SOIN CORPS: BODY CARE SC028, FORMULA A056 présente une diminution de 9,7% et de 15,5% de l'épaisseur du tissu adipeux, après 14 et 28 jours d'application, respectivement. Ces changements sont statistiquement significatifs.

Le produit SOIN CORPS: BODY CARE SC028, FORMULA A056 a présenté une amélioration de 18,4% et de 20,2% de la fermeté de la peau, après 14 et 28 jours d'application, respectivement. Ces changements sont statistiquement significatifs.

Le produit SOIN CORPS: BODY CARE SC028, FORMULA A056 a présenté une augmentation de 6,4% et de 11,2% de l'élasticité de la peau, après 14 et 28 jours d'application, respectivement. Ces changements sont statistiquement significatifs.

Le produit SOIN CORPS: BODY CARE SC028, FORMULA A056 a présenté une diminution de 1,3% et de 2,7% des valeurs centimétriques de la cuisse, après 14 et 28 jours d'application, respectivement. Ces changements sont statistiquement significatifs.

### 2/ Scores cliniques évalué par le dermatologue :

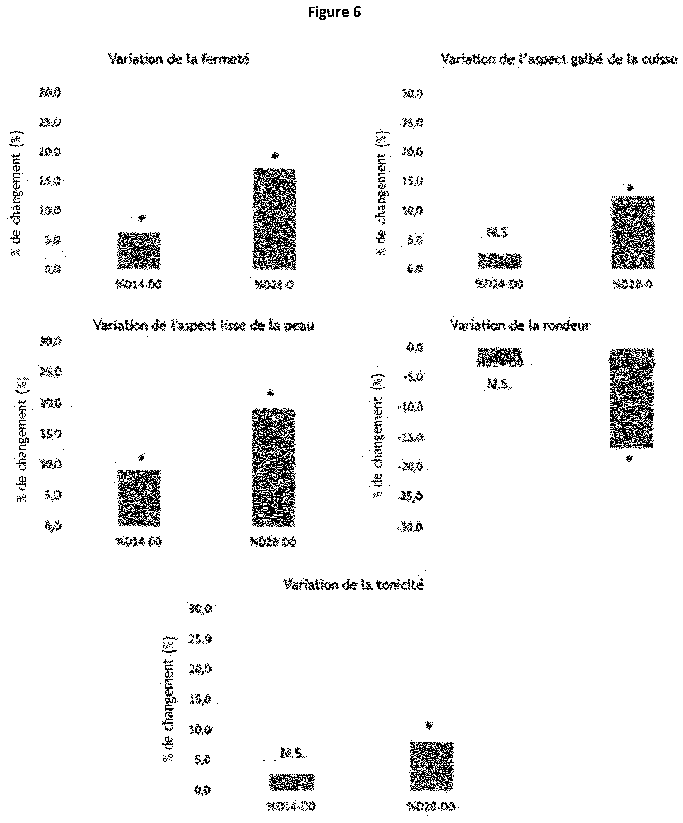

Les résultats sont présentés sur la figure 6.

Les autres paramètres ont été évalués de la même façon (non montré). En résumé : Le produit SOIN CORPS: BODY CARE SC028, FORMULA A056 a présenté une amélioration de 0,5% et de 4,2% du score final (CSS = A + B + C + D + E), après 14 et 28 jours d'application, respectivement. Ces changements sont statistiquement significatifs après 28 jours.

Le produit SOIN CORPS: BODY CARE SC028, FORMULA A056 a présenté une amélioration de 6,4% et de 17,3% de la fermeté, après 14 et 28 jours d'application, respectivement. Ces changements sont statistiquement significatifs après 14 et 28 jours.

Le produit SOIN CORPS: BODY CARE SC028, FORMULA A056 a présenté une amélioration de 2,7% et de 12,5% de l'aspect galbé de la cuisse, après 14 et 28 jours d'application, respectivement. Ces changements sont statistiquement significatifs après 28 jours.

Le produit SOIN CORPS: BODY CARE SC028, FORMULA A056 a présenté une amélioration de 9,1% et de 19,1% de l'aspect lisse de la peau, après 14 et 28 jours d'application, respectivement. Ces changements sont statistiquement significatifs après 14 et 28 jours.

Le produit SOIN CORPS: BODY CARE SC028, FORMULA A056 a présenté une amélioration de 2,5% et de 16,7% des rondeurs, après 14 et 28 jours d'application, respectivement. Ces changements sont statistiquement significatifs après 28 jours.

Le produit SOIN CORPS: BODY CARE SC028, FORMULA A056 a présenté une amélioration de 2,7% et de 8,2% de la tonicité, après 14 et 28 jours d'application, respectivement. Ces changements sont statistiquement significatifs après 28 jours.

Les résultats suggèrent que le produit SOIN CORPS: BODY CARE SC028, FORMULA A056 montre une capacité significative à redéfinir la forme de la cuisse, l'amincir, tout en améliorant la fermeté de la peau de la cuisse et l'apparence générale de la cellulite. De plus, ces résultats montrent que le produit SOIN CORPS: BODY CARE SC028, FORMULA A056 a une influence significative sur le galbe de la cuisse et sa qualité de peau (aspect de la cellulite, fermeté, tonicité, aspect lisse de la peau) selon le point de vue du dermatologue.

### 3/ Score clinique évalué par les sujets

Le produit SOIN CORPS: BODY CARE SC028, FORMULA A056 a présenté une amélioration de 12,9% et de 34,7% de la fermeté, après 14 et 28 jours d'application. Ces changements sont statistiquement significatifs après 14 et 28 jours.

Le produit SOIN CORPS: BODY CARE SC028, FORMULA A056 a présenté une amélioration de 11,1% et de 24,1% de l'aspect galbé de la cuisse, après 14 et 28 jours d'application. Ces changements sont statistiquement significatifs après 14 et 28 jours.

Le produit SOIN CORPS: BODY CARE SC028, FORMULA A056 a présenté une amélioration de 16,3% et de 24,4% de l'aspect lisse de la peau, après 14 et 28 jours d'application. Ces changements sont statistiquement significatifs après 14 et 28 jours.

Le produit SOIN CORPS: BODY CARE SC028, FORMULE A056 a présenté une amélioration de 4,5% et de 15,0% des rondeurs, après 14 et 28 jours d'application. Ces changements sont statistiquement significatifs après 14 et 28 jours.

Le produit SOIN CORPS: BODY CARE SC028, FORMULE A056 a présenté une amélioration de 9,3% et de 24,0% de l'inconfort au pincement, après 14 et 28 jours d'application. Ces changements sont statistiquement significatifs après 28 jours.

Le produit SOIN CORPS: BODY CARE SC028, FORMULA A056 a présenté une amélioration de 5,0% et de 18,5% de l'aspect lisse de la cellulite au pincement, après 14 et 28 jours d'application. Ces changements sont statistiquement significatifs après 14 et 28 jours.

Le produit SOIN CORPS: BODY CARE SC028, FORMULA A056 a présenté une amélioration de 3,7% et de 22,2% de l'aspect de la cellulite, après 14 et 28 jours d'application. Ces changements sont statistiquement significatifs après 28 jours.

### 4/ Résultats du questionnaire :

Le produit SOIN CORPS: BODY CARE SC028, FORMULA A056 a été bien apprécié par les sujets en ce qui concerne les questions suivantes:

### Beauté de la peau à J14 :

La peau est plus nourrie / La peau est plus confortable / La peau est plus douce / plus veloutée

### Texture / application à J0 immédiat:

Le produit procure une sensation de fraîcheur et de légèreté lors de l'application / Texture non grasse / Le produit / la texture permet de s'habiller rapidement après application / Le produit s'étale facilement Le produit / la texture convient au massage

### Action anti-cellulite à J14:

### Les capitons sont visiblement lissés

Galbe et tonicité à J14:

### La peau est plus ferme / La peau est comme gainée

### Action anti-cellulite à J28 :

### Les capitons sont visiblement lissés / L'aspect des capitons au pincement est amélioré

### Galbe et tonicité à J28 :

La peau est plus ferme / La peau est plus tonique / La peau est comme gainée

### Beauté de la peau à J28 :

La peau est plus lisse / La peau est plus nourrie /La peau est plus confortable / La peau est plus douce / plus veloutée / La qualité de la peau est améliorée / Le produit améliore visiblement la beauté de la peau

### Texture / application à J28 :

Le produit procure une sensation de fraîcheur et de légèreté lors de l'application / Texture non grasse / Le produit / la texture permet de s'habiller rapidement modifier l'application / Le produit s'étale facilement / Le produit / la texture convient au massage

Les résultats suggèrent que le produit SOIN CORPS: BODY CARE SC028, FORMULA A056 montre une capacité significative à redéfinir le galbe de la cuisse, l'amincir, tout en améliorant la fermeté de la peau de la cuisse et l'apparence générale de la cellulite. De plus, ces résultats montrent qu'il a une influence significative sur le galbe de la cuisse et sur la qualité de peau (aspect de la cellulite, fermeté, aspect lisse de la peau) ainsi que sur la sensation d'inconfort sur les zones avec cellulite selon le point de vue du sujet.

Plus précisément, le tableau 2 décrit le nombre de réponses positives fournies par les sujets au sujet de l'action anti-cellulite du produit de l'invention à J14.

| **Questions sur l'action anti-cellutite** | | |
|---|---|---|
| | **Score Moyen** | **J14 % de sujets satisfaits** |
| Q1 Les capitons sont visiblement lissés | 2,90 | 76,2 |

Et le tableau 3 décrit le nombre de réponses positives fournies par les sujets au sujet de l'action anti-cellulite du produit testé à J28.

| **Questions sur l'action anti-cellulite** | **J28** | |
|---|---|---|
| | **Score Moyen** | **% de sujets satisfaits** |
| Q1. Les capitons sont visiblement lissés | 3,05 | 81,0 |
| Q6. L'aspect des capitons est atténué | 2,86 | 66,7 |
| Q7. L'aspect des capitons au pincement est amélioré | 3,00 | 81,0 |
| Q4. Les sensations d'inconfort au pincement sont réduites | 2,86 | 66,7 |
| 45. L'aspect peau d'orange est atténué application après application | 2,52 | 66,7 |

### D. Exemple d'une composition cosmétique selon l'invention

Un produit selon l'invention contient par exemple :

| **FORMULE:** | | | |
|---|---|---|---|
| **N° CAS** | **Ingrédients INCI** | **Fonction** | **% w/w** |
| 7732-18-5 | EAU | *SOLVANTS* | qsp 100 |
| 64-17-5 | ALCOOL DENAT. | *SOLVANTS* | 15 |
| 5343-92-0 | PENTYLENE GLYCOL | *SOLVANTS* | 2 |
| 107-88-0 | BUTYLENE GLYCOL | *SOLVANTS* | 5 |
| 194043-92-0 | Beurre de Karité BUTYROSPERMUM PARKII | *AGENTS CONDITIONNANTS DE LA PEAU* - *DIVERS* | 1 |
| 58-08-2 | CAFEINE | *AGENTS CONDITIONNANTS DE LA PEAU* - *DIVERS* | 3 |
| 68201-46-7 | COCOATE DE GLYCERYLE PEG-7 | *AGENTS CONDITIONNANTS DE LA PEAU* - *DIVERS* | 4 |
| 73398-61-5 | TRIGLYCERIDE CAPRYLIQUE/CAPRIQUE | *AGENTS CONDITIONNANTS DE LA PEAU* - *OCCLUSIF* | 1 |
| 111286-86-3 | HYDROXYETHYL ACRYLATE/SODIUM ACRYLOYLDIMETHYL TAURATE COPOLYMER | *AGENTS D'AUGMENTATION DE LA VISCOSITÉ* - *AQUEUX* | 1,7 |
| 352458-32-3 | Huile de graine de CARAPA GUAIANENSIS (huile d'Andiroba) | *AGENTS CONDITIONNANTS DE LA PEAU* - *DIVERS* | 1 |
| 56-81-5 | GLYCERINE | *AGENTS CONDITIONNANTS DE LA PEAU* - *HUMECTANT* | 3 |
| 84929-38-4 | Extrait de fruit CITRUS NOBILIS (MANDARIN ORANGE) | *AGENTS CONDITIONNANTS DE LA PEAU* - *DIVERS* | 1 |
| 8000-28-0 | Huile essentielle de LAVANDULA ANGUSTIFOLIA (LAVENDE) | *INGREDIENTS PARFUMANTS* | 0.12 |
| 84837-14-9 (EU) | Huile essentielle de fleur THYMUS MASTICHINA | *INGREDIENTS PARFUMANTS* | 0.12 |
| 8000-25-7 | Huile essentielle de feuille ROSMARINUS OFFICINALIS (ROMARIN) | *INGREDIENTS PARFUMANTS* | 0.08 |
| 8022-56-8 | Huile essentielle de SALVIA OFFICINALIS (SAUGE) | *INGREDIENTS PARFUMANTS* | 0.08 |
| 29806-73-3 | PALMITATE D'ETHYLHEXYL | *AGENTS CONDITIONNANTS DE LA PEAU* - *EMOLLIENT* | 2 |
| 9005-67-8 | POLYSORBATE 60 | *SURFACTANTS* - *AGENTS EMULSIFIANTS* | 0,12 |
| 54392-26-6 71902-01-7 | ISOSTEARATE DE SORBITAN | *SURFACTANTS* - *AGENTS EMULSIFIANTS* | 0,12 |
| 5949-29-1 77-92-9 | ACIDE CITRIQUE | *AJUSTEUR DE PH* | 0.04 |
| 11138-66-2 | GOMME DE XANTHANE | *AGENTS D'AUGMENTATION DE LA VISCOSITÉ* - *AQUEUX* | 0,0200 |
| 1310-73-2 | HYDROXYDE DE SODIUM | *AJUSTEUR DE PH* | 0,0050 |
| 10191-41-0 | TOCOPHEROL | *ANTIOXIDANTS* | 0,0020 |
| | TOTAL | | 100,0000 |

## Revendications

1. Utilisation topique d'une composition cosmétique comprenant une quantité efficace d'au moins un inhibiteur de l'enzyme de transfert des céramides (CERT) pour prévenir ou diminuer la cellulite, et/ou pour promouvoir un effet amincissant.

2. Utilisation selon la revendication 1, **caractérisée en ce que** ledit inhibiteur de l'enzyme CERT est choisi parmi : HPA-12, la cedrelone, la khayanthone, l'odoratone, la gédunine, la khivorine, l'isogedunine, la limonine, la methylangolensate, l'obliquine, la prieuranine, la carapine ou l'un de leurs dérivés.

3. Utilisation selon la revendication 1 ou 2, **caractérisée en ce que** ladite composition cosmétique comprend une quantité efficace de gédunine et/ou une quantité efficace de limonine ou d'un de leur sel ou dérivé acceptable.

4. Utilisation selon la revendication 3, **caractérisée en ce que** ladite composition cosmétique contient au moins 0.01 ppm de limonine et/ou d'un de ses dérivés et/ou au moins 5 ppm de gédunine et/ou d'un de ses dérivés, de préférence au moins 0.04 ppm de limonine et/ou d'un de ses dérivés et/ou au moins 9 ppm de gédunine et/ou d'un de ses dérivés.

5. Utilisation selon l'une quelconque des revendications 1 à 4, **caractérisée en ce que** ladite composition cosmétique contient au moins 0,5 % d'un extrait de plante contenant au moins 1000ppm de gédunine et/ou d'un de ses dérivés ou au moins 0,5 % d'un extrait de plante contenant au moins 4 ppm, de préférence 15 ppm, de limonine et/ou d'un de ses dérivés.

6. Utilisation selon l'une quelconque des revendications 1 à 5, **caractérisée en ce que** ladite composition cosmétique contient au moins 1% d'un extrait de plante contenant au moins 1000ppm de gédunine et/ou d'un de ses dérivés et au moins 1 % d'un extrait de plante contenant au moins 4 ppm, de préférence 15 ppm, de limonine et/ou d'un de ses dérivés.

7. Utilisation selon la revendication 5 ou 6, **caractérisée en ce que** ladite composition cosmétique comprend une quantité efficace d'un extrait d'Andiroba et une quantité efficace d'un extrait de mandarine.

8. Utilisation selon la revendication 7, **caractérisée en ce que** ladite composition cosmétique comprend au moins 0,5 % d'huile d'Andiroba contenant au moins 1000ppm de gédunine et/ou d'un de ses dérivés et au moins 0,5 % d'un extrait de mandarine contenant au moins 4 ppm, de préférence 15 ppm, de limonine et/ou d'un de ses dérivés.

9. Utilisation selon la revendication 7, **caractérisée en ce que** ladite composition cosmétique comprend au moins 1 % d'huile d'Andiroba contenant au moins 1000ppm de gédunine et/ou d'un de ses dérivés et au moins 1 % d'un extrait de mandarine contenant au moins 4 ppm, de préférence 15 ppm, de limonine et/ou d'un de ses dérivés.

10. Composition cosmétique à usage topique, comprenant une quantité efficace d'huile d'Andiroba et d'un extrait de mandarine.

11. Composition cosmétique à usage topique selon la revendication 10, contenant au moins 0.01 ppm de limonine et au moins 5 ppm de gédunine et/ou d'un de ses dérivés, de préférence au moins 0.04 ppm de limonine et au moins 9 ppm de gédunine et/ou d'un de ses dérivés.

12. Composition cosmétique à usage topique selon la revendication 10, comprenant au moins 0,5 % d'huile d'Andiroba contenant au moins 1000ppm de gédunine et/ou d'un de ses dérivés et au moins 0,5 % d'un extrait de mandarine contenant au moins 4 ppm, de préférence 15 ppm, de limonine et/ou d'un de ses dérivés.

13. Méthode de traitement cosmétique de la cellulite, comprenant l'application de la composition décrite dans les revendications 10 à 12 sur la peau d'un individu sain.
